# EUROPEAN PATENT APPLICATION

(11) **EP 4 342 982 A1**
(43) Date of publication of application: **27.03.2024**
(21) Application number: 22825104.7
(22) Date of filing: 17.06.2022
(51) Int. Cl.: C12N 5/078, A61K 35/19, A61P 7/04, C12N 5/10, C12N 15/09

(54) **METHOD FOR PRODUCING MULTINUCLEATED MEGAKARYOCYTE WITH ENHANCED PLATELET PRODUCTION CAPABILITY, METHOD FOR PRODUCING PLATELETS, METHOD FOR PRODUCING PLATELET PREPARATION, AND METHOD FOR PRODUCING BLOOD PREPARATION**

(30) Priority: 18.06.2021 JP 2021102068
(71) Applicant: Megakaryon Corporation, Kyoto-shi, Kyoto 600-8813 (JP)
(72) Inventor: ITO, Yukitaka, Kyoto-shi, Kyoto 600-8813 (JP); MISUMI, Sachiyo, Kyoto-shi, Kyoto 600-8813 (JP)
(74) Representative: Schön, Christoph
(86) International application number: PCT/JP2022/024432
(87) International publication number: WO 2022/265117

(57) **Abstract**

A method for improving the amount of platelets produced. The production method is a method for producing multinucleated megakaryocyte cells with enhanced platelet production capability, the method including a multinucleation step of inducing multinucleated megakaryocyte cells through multinucleation of pre-multinucleated megakaryocyte cells in the presence of a growth factor of the pre-multinucleated megakaryocyte cells. In the multinucleation step, the multinucleated megakaryocyte cells are induced by suppressing the activity of at least one protein selected from the group consisting of an aryl hydrocarbon receptor (AhR), a Rho-associated kinase (ROCK), dual-specificity tyrosine phosphorylation-regulated kinases (DYRK), and myosin 2, and multinucleating the pre-multinucleated megakaryocyte cells in the presence of harmine.

## Description

### TECHNICAL FIELD

The present invention relates to a method for producing a multinucleated megakaryocyte cell with enhanced platelet production capability, a method for producing platelets, a method for producing a platelet preparation, and a method for producing a blood preparation.

### BACKGROUND ART

Platelet preparations are administered to patients associated with a reduction in platelet count during bleeding due to surgery, injury, and the like. In recent years, platelet preparations are produced from donated blood. However, due to demographic changes, there are concerns that the amount of donated blood will decrease, which results in a shortage of platelet preparations.

Also, when a blood donor has a bacterial infection or the like, the blood may be contaminated with bacteria, and thus there is a risk of infection from administration of platelet preparations contaminated with bacteria. Therefore, methods have been developed to produce platelets *in vitro* (Non Patent Literature 1).

### Citation List

### Non Patent Literature

Non Patent Literature 1: Takayama N et.al, "Generation of functional platelets from human embryonic stem cells in vitro via ES-sacs, VEGF-promoted structures that concentrate hematopoietic progenitors.", 2008, Blood, Vol. 111, No. 11, pages 5298-5306

### SUMMARY OF INVENTION

### Technical Problem

However, when the method disclosed in Non Patent Literature 1 is used to produce platelets in an amount that are normally administered at one time to a human, there is a problem that a huge production tank is necessary.

Megakaryocyte cells prior to multinucleation (hereinafter, referred to as "pre-multinucleated megakaryocytes") produce platelets after multinucleation. Specifically, pre-multinucleated megakaryocytes expand cytoplasm through multinucleation. Then, the resulting multinucleated megakaryocyte cells (also referred to as "multinucleated megakaryocytes", hereinafter) undergo rupture of expanded cytoplasm, thereby producing platelets. In view of this, the inventors of the present invention attempted to improve the platelet production capability of pre-multinucleated megakaryocyte cells, through facilitation of multinucleation and expansion associated with multinucleation. However, the inventors of the present invention found that, even when multinucleation and expansion occur, the platelet production capability of the obtained multinucleated megakaryocytes is not always improved. It is presumed that this is because the maximum platelet production capability is improved through facilitation of multinucleation and expansion of megakaryocyte cells, but the differentiation and maturation of megakaryocyte cells do not sufficiently progress, and as a result, platelet release capability is not improved.

In view of this, the present invention aims to provide a method for producing multinucleated megakaryocytes with enhanced platelet production capability.

### Solution to Problem

In order to achieve the above aim, a production method of the present invention (also referred to as a "method for producing a multinucleated megakaryocyte" hereinafter) is a method for producing multinucleated megakaryocyte cells with enhanced platelet production capability, and the method includes: a multinucleation step of inducing multinucleated megakaryocyte cells through multinucleation of pre-multinucleated megakaryocyte cells or progenitor cells thereof in the presence of a growth factor of the pre-multinucleated megakaryocyte cells, wherein the multinucleation step, multinucleated megakaryocyte cells are induced by suppressing the activity of at least one protein selected from the group consisting of an aryl hydrocarbon receptor (AhR), a Rho-associated kinase (ROCK), dual-specificity tyrosine phosphorylation-regulated kinases (DYRK), and myosin 2, and multinucleating the pre-multinucleated megakaryocyte cells in the presence of harmine.

A method for producing platelets according to the present invention includes: a multinucleation step of inducing multinucleated megakaryocyte cells from pre-multinucleated megakaryocyte cells; and a platelet production step of producing platelets from the multinucleated megakaryocyte cells, where the multinucleation step is carried out using the method for producing multinucleated megakaryocyte cells according to the present invention.

A method for producing a platelet preparation according to the present invention includes: a preparation step of producing a platelet preparation from platelets, where the platelets are obtained using the method for producing platelets according to the present invention.

A method for producing a blood preparation according to the present invention includes a blood preparation step of producing a blood preparation by mixing platelets with another component,
where the platelets are obtained using the method for producing platelets according to the present invention.

A cell population according to the present invention is a cell population containing multinucleated megakaryocytes,
the multinucleated megakaryocytes include multinucleated megakaryocytes having a nuclear phase of 16N or more, and
a percentage (number of cells) of the multinucleated megakaryocytes having a nuclear phase of 16N or more in the cell population is 20% or more.

A cell population according to the present invention is a cell population containing multinucleated megakaryocytes,
where the multinucleated megakaryocytes include multinucleated megakaryocytes having a nuclear phase of 32N or more, and
a percentage (number of cells) of the multinucleated megakaryocytes having a nuclear phase of 32N or more in the cell population is in a range of 5% to 50%.

### Advantageous Effects of Invention

According to the present invention, it is possible to produce multinucleated megakaryocytes with enhanced platelet production capability.

### BRIEF DESCRIPTION OF DRAWINGS

[FIG. 1] FIG. 1 shows graphs of the degree of multinucleation in Example 1.
[FIG. 2] FIG. 2 shows a graph illustrating the amount of platelets produced per megakaryocyte in Example 1.
[FIG. 3A] FIG. 3A shows graphs illustrating the degree of multinucleation and the size of cells in Reference Example 1A.
[FIG. 3B] FIG. 3B shows graphs illustrating the degree of multinucleation and the size of cells in Reference Example 1B.
[FIG. 3C] FIG. 3C shows graphs illustrating the degree of multinucleation and the size of cells in Example 1.
[FIG. 4] FIG. 4 shows photographs of phase-contrast images of cells in an F1 fraction in Example 1.
[FIG. 5] FIG. 5 shows graphs illustrating the amount of platelets produced per megakaryocyte in Example 1.
[FIG. 6] FIG. 6 shows a graph illustrating the amount of platelets produced per megakaryocyte in Example 2.
[FIG. 7] FIG. 7 shows graphs illustrating a nuclear phase of multinucleated megakaryocytes in Example 3.

### DESCRIPTION OF EMBODIMENTS

### Method for producing multinucleated megakaryocytes

As described above, the method for producing multinucleated megakaryocytes according to the present invention is a method for producing multinucleated megakaryocyte cells with enhanced platelet production capability. The method includes a multinucleation step of inducing multinucleated megakaryocyte cells through multinucleation of pre-multinucleated megakaryocytes or progenitor cells thereof in the presence of a growth factor of the pre-multinucleated megakaryocyte cells, where, in the multinucleation step, the multinucleated megakaryocytes are induced by suppressing the activity of at least one protein selected from the group consisting of an aryl hydrocarbon receptor (AhR), a Rho-associated kinase (ROCK), dual-specificity tyrosine phosphorylation-regulated kinases (DYRK), and myosin 2, and multinucleating the pre-multinucleated megakaryocyte cells in the presence of harmine. According to the present invention, the platelet production capability of the obtained multinucleated megakaryocyte cells can be enhanced by suppressing the activity of at least one protein selected from the group consisting of AhR, ROCK, DYRK, and myosin 2, and multinucleating the pre-multinucleated megakaryocyte cells in the presence of harmine.

The method for producing multinucleated megakaryocyte cells with enhanced platelet production capability according to the present invention may also be referred to as a method for producing multinucleated megakaryocyte cells with advanced differentiation or improved maturity. Also, multinucleation of megakaryocytes can be facilitated using the method for producing multinucleated megakaryocyte cells according to the present invention, and thus the method for producing multinucleated megakaryocyte cells according to the present invention may also be referred to as a method for facilitating multinucleation of megakaryocyte cells or a method for enhancing a nuclear phase of megakaryocyte cells.

In the present invention, the term "enhancement" indicates that a desired parameter is significantly increased, compared to that of a control group. Therefore, the enhancement may also be referred to as, for example, increasing, improving, or strengthening.

The platelet production capability can be enhanced using the method for producing multinucleated megakaryocytes according to the present invention. As a result, the amount of platelets produced can be increased, for example, and thus, the method for producing multinucleated megakaryocytes according to the present invention may also be referred to as a method for enhancing platelet production or the amount of platelets produced, a method for improving platelet production or the amount of platelets produced, a method for ameliorating platelet production or the amount of platelets produced, or the like. The method for producing multinucleated megakaryocytes according to the present invention, for example, is a method carried out *in vitro.*

In the present invention, a "megakaryocyte cell (megakaryocyte)" refers to the largest type of the cells present in the bone marrow in an organism, and refers to a cell that can release platelets, a cell that releases platelets, and a cell having an equivalent function. The cell having the equivalent function refers to a cell having a capability to produce platelets. In the present invention, megakaryocytes may be pre-multinucleated (polyploid) megakaryocytes, that is, immature megakaryocytes or proliferative megakaryocytes, or post-multinuclear megakaryocytes (multinucleated megakaryocytes). As a specific example, the megakaryocyte may be, for example, a promegakaryoblast, a megakaryoblast, a promegakaryocyte, or a mature megakaryocyte. The number of sets of chromosomes in the multinucleated megakaryocyte (nuclear phase: N) need only exceed 2 sets, and specific examples thereof include 4 to 64 sets (4N to 64N), 4 to 32 sets (4N to 32N), 8 to 64 sets (8 to 64N), 16 to 32 sets (16 to 32N), or 16 to 64 sets (16 to 64N).

There is no particular limitation on the origin of the megakaryocytes, and examples thereof include humans and non-human animals. Examples of the non-human animals include primates such as monkeys, gorillas, chimpanzees, and marmosets, and the like, and mice, rats, dogs, cats, rabbits, sheep, horses, and guinea pigs, and the like.

In the present invention, the megakaryocytes can be identified by cell surface markers. If the megakaryocytes are derived from a human, examples of the cell surface marker include CD41a, CD42a, and CD42b. That is, the megakaryocytes are cells positive for CD41a, CD42a, and CD42b. If the megakaryocytes are derived from a human, the cell surface marker may be, for example, at least one cell surface marker selected from the group consisting of CD9, CD61, CD62p, CD42c, CD42d, CD49f, CD51, CD 110, CD 123, CD 131, and CD203c.

The pre-multinucleated megakaryocytes and the multinucleated megakaryocytes can be, for example, specified by a number of sets of chromosomes. As a specific example, when the number of sets of chromosomes (N) in a target megakaryocyte cell is less than 8N, the target megakaryocyte cell can be evaluated to be a pre-multinucleated megakaryocyte. On the other hand, when the number of sets of chromosomes (N) in a target megakaryocyte cell is 8N or more, or preferably 16N or more, the target megakaryocyte cell can be evaluated to be a multinucleated megakaryocyte. The number of sets of chromosomes (nuclear phase) can be evaluated according to Example 1(3) described later.

In the case of a plurality of the target megakaryocyte cells, that is, in the case of a population of the target megakaryocyte cells (cell population), when a percentage of cells having a number of sets of chromosomes (N) of 8N or more in the target megakaryocyte cell population is less than 10%, the target megakaryocyte cell population can be evaluated to be a population of pre-multinucleated megakaryocytes. Further, in the target megakaryocyte cell population, when the percentage of cells having a number of sets of chromosomes (N) of 8N or more is 20% or more, 21% or more, 22% or more, 23% or more, 24% or more, 25% or more, 26% or more, 27% or more, 28% or more, 29% or more, 30% or more, 35% or more, 40% or more, 45% or more, or 50% or more, the target megakaryocyte cell population can be evaluated to be a population containing multinucleated megakaryocytes. The target megakaryocyte cell population, for example, includes from 1×10⁵ to 1×10⁶ cells.

The "platelet production capability" in the present invention can be evaluated, for example, as the number of platelets based on the number of megakaryocyte cells used for platelet production. Examples of the megakaryocyte cells include pre-multinucleated megakaryocytes, multinucleated megakaryocytes, and mature megakaryocytes. The "platelet production capability" can be, for example, comparatively examined by culturing approximately the same number of megakaryocyte cells using megakaryocyte cells at approximately the same differentiation, maturation, or culture stage. As a specific example, the "platelet production capability" can be, for example, expressed as the ratio of the number of platelets (P) after the platelet production step to the number of multinucleated megakaryocytes (M) at the start of platelet production (P/M ratio). In this case, the improvement of the platelet production capability of the multinucleated megakaryocytes means, for example, that without suppressing the activities of AhR, ROCK, DYRK, and myosin 2, and in the absence of harmine, the P/M ratio of the multinucleated megakaryocytes, which were obtained by multinucleating the pre-multinucleated megakaryocyte cells, is significantly high. As will be described later, when the production of platelets is started using immortalized megakaryocytes, the number of multinucleated megakaryocytes (M) at the start of platelet production may be replaced with the number of immortalized megakaryocytes (M) when the forced expression of a gene is halted, and a description of the P/M ratio can be applied thereto.

The "platelet" in the present invention is one type of the cell components in blood, and refers to a cell component that is positive for CD41a and CD42b. The platelet, for example, does not have a cell nucleus, and the size of the platelet is smaller than that of a megakaryocyte. Thus, the platelets and the megakaryocytes can be, for example, distinguished by the presence/absence of cell nuclei and/or the size. It is known that the platelets play an important role in thrombus formation and hemostasis, and are also involved in the pathophysiology of tissue regeneration and inflammation after injury. Further, it is known that, if the platelets are activated due to bleeding or the like, receptors for cell adhesion factors, such as Integrin αIIBβ3 (glycoprotein IIb/IIIa; a complex of CD41a and CD61), are expressed on the platelet membrane. Also, if the platelets are activated, the platelets aggregate, and fibrin coagulates due to various blood coagulation factors released from the platelets. Therefore, a thrombus is formed, and hemostasis progresses. In the present invention, the origin of the platelets is the same as the origin of the megakaryocytes.

In the present invention, the physiological activity of platelets can be evaluated using a known method. The physiological activity of the platelets can be, for example, evaluated by evaluating the amount of activated platelets using an antibody against PAC-1, which specifically binds to Integrin αIIBβ3 on the activated platelet membrane. Also, the physiological activity of the platelets may be, for example, evaluated by detecting CD62p (P-selectin), which is an activation marker for platelets, using an antibody, and evaluating the amount of activated platelets. The physiological activity of the platelets may be, for example, evaluated by performing flow cytometry using an antibody against activation-independent platelet marker CD61 or CD41 for gating, and then detecting the binding of anti-PAC-1 antibody or anti-CD62p antibody. The physiological activity of platelets may be evaluated in the presence of adenosine diphosphate (ADP).

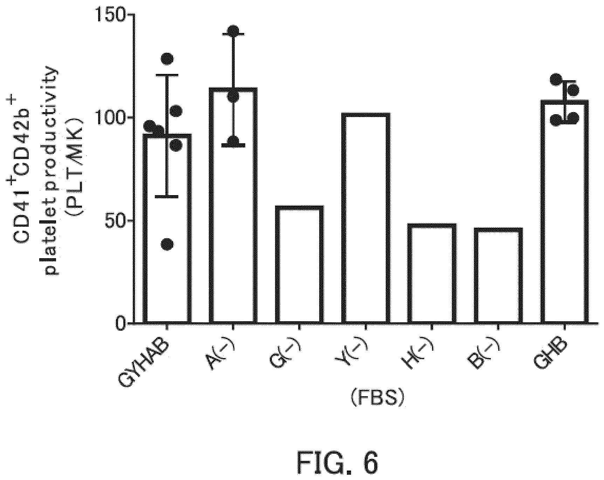

In the present invention, the physiological activity of platelets may be, for example, evaluated by checking whether or not platelets bind to fibrinogen in the presence of ADP. When the platelets bind to fibrinogen, integrins that are necessary for the initial stage of thrombus formation are activated. Further, for example, as shown in FIG. 6 in WO 2011/034073, which is herein incorporated by reference, the physiological activity of platelets may be evaluated using a method in which thrombus formation capability is visualized and observed *in vivo.*

The wording "high functionality (high physiological activity)" in the present invention means, for example, that the physiological activity of platelets measured using any one of the above-described methods is significantly higher than or tends to be higher than that of platelets obtained using a conventional method, or equivalent to the physiological activity of platelets isolated from an organism. Also, the wording "high functionality" may indicate, for example, that the physiological activity of platelets measured using any one of the above-described methods is 50% or more, 60% or more, 70% or more, 80% or more, or 90% or more of the physiological activity of platelets isolated from an organism.

When the expression rate of CD42b in the platelets is low, or when the annexin V positive rate is low, for example, the platelets can be evaluated to be degraded or abnormal (also collectively referred to as "degradation" hereinafter), that is, the physiological activity of the platelets can be evaluated to be low. Therefore, as the percentage of degraded platelets in the produced platelets decreases, for example, the physiological activity of the produced platelets can be evaluated to be higher. Also, degraded platelets do not have sufficient thrombus formation function (blood coagulation function) or hemostasis function, for example, and have low clinical usefulness.

The "degradation of platelets" in the present invention, for example, refers to a decrease in CD42b (GPIbα) on the surfaces of the platelets. That is, degraded platelets include, for example, platelets with reduced CD42b expression, and platelets in which an extracellular region of CD42b is cleaved by a shedding reaction. When CD42b is not present on the platelet surface, the platelets cannot associate with, for example, von Willebrand factor (VWF), and as a result, the blood coagulation function of the platelets will be lost. Degradation of the platelets can be, for example, evaluated using, as an index, the CD42b negative rate (or the number of CD42b negative particles) relative to the CD42b positive rate (or the number of CD42b positive particles) in a fraction of the platelets. As a specific example, the higher the CD42b negative rate relative to the CD42b positive rate is, or the greater the number of CD42b negative particles relative to the number of CD42b positive particles is, the more degraded the platelets can be evaluated to be. The CD42b positive rate refers to the percentage of platelets to which an anti-CD42b antibody can bind, out of the platelets contained in the platelet fraction. Also, the CD42b negative rate refers to the percentage of platelets to which an anti-CD42b antibody does not bind, out of the platelets contained in the platelet fraction.

An "abnormal platelet" in the present invention refers to, for example, a platelet in which phosphatidylserine,that is a negatively charged phospholipid, is exposed from the inside of a lipid bilayer to the outside. It is known that, in an organism, phosphatidylserine is exposed on a surface accompanying the activation of platelets, and a large number of blood coagulation factors bind thereto, as a result of which a blood coagulation cascade reaction is amplified. On the other hand, a large amount of phosphatidylserine is always exposed on surfaces of the abnormal platelets, for example, and therefore, when the abnormal platelets are administered to a patient, an excessive blood coagulation reaction occurs, which may lead to serious pathological conditions such as disseminated intravascular coagulation syndrome. As it is known that annexin V binds to phosphatidylserine, phosphatidylserine on the platelet surface can be detected, for example, by using a flow cytometer, using, as an indicator, the amount of bound fluorescently labeled annexin V Therefore, the amount of the abnormal platelets can be, for example, evaluated using the annexin V positive rate in the platelet fraction, that is, the percentage or number of platelets to which annexin V binds. As a specific example, as the annexin V positive rate increases, or as the number of annexin V particles increases, it can be evaluated that target platelets include a larger number of abnormal platelets.

In the present invention, the pre-multinucleated megakaryocytes can be, for example, induced from cells that are less differentiated than the megakaryocytes (also referred to as "progenitor cells of pre-multinucleated megakaryocytes" hereinafter). Therefore, the method for producing multinucleated megakaryocytes according to the present invention, for example, may include a megakaryocyte induction step of inducing pre-multinucleated megakaryocytes from cells that are less differentiated than the megakaryocytes, prior to the multinucleation step. For example, the description of a later-described multinucleation step can be applied to a culture medium, culture conditions, and the like used in the megakaryocyte induction step.

The "cells that are less differentiated than megakaryocytes" refers to cells having the capability to differentiate into the megakaryocytes. Specific examples of the cells that are less differentiated than megakaryocytes include hematopoietic stem cells, hematopoietic progenitor cells, CD34-positive cells, megakaryocyte-erythroid progenitors (MEPs), and megakaryocyte progenitor cells. The cell that is less differentiated than megakaryocytes may be isolated from bone marrow, umbilical cord blood, peripheral blood, or the like, or may be induced from pluripotent cells or pluripotent stem cells such as ES cells (embryonic stem cells), induced pluripotent stem cells (iPS cells), nuclear transfer ES cells (ntES cells), reproductive stem cells, somatic stem cells, and embryonic tumor cells.

There is no particular limitation on the method for inducing the megakaryocytes, and the megakaryocytes can be induced using a known induction method. Specific examples of the method for inducing the megakaryocytes include the methods disclosed in WO 2011/034073 and WO 2012/157586. As a specific example, in the megakaryocyte induction step, the oncogene and the Polycomb gene may be forcibly expressed in, for example, the cells that are less differentiated than the megakaryocytes. As a result, in the megakaryocyte induction step, for example, it is possible to obtain immortalized megakaryocytes that correspond to pre-multinucleated megakaryocytes and proliferate infinitely. Further, for example, by halting the forced expression of the immortalized megakaryocytes, the immortalized megakaryocytes can be induced into multinucleated megakaryocytes to produce platelets. Therefore, the immortalized megakaryocytes correspond to pre-multinucleated megakaryocytes. Also, in the megakaryocyte induction step, for example, the apoptosis suppressor gene may be forcibly expressed in the megakaryocyte progenitor cells. As a result, it is possible to obtain the immortalized megakaryocytes in the megakaryocyte induction step. Further, in a later-described platelet production step, for example, by halting the forced expression of the immortalized megakaryocytes, the immortalized megakaryocytes become mature and the platelets are produced.

In the megakaryocyte induction step, for example, the oncogene, the Polycomb gene, and the apoptosis suppressor gene may also be forcibly expressed. In this case, the oncogene, the Polycomb gene, and the apoptosis suppressor gene may also be forcibly expressed simultaneously or separately. As specific examples, in the megakaryocyte induction step, after the oncogene and the Polycomb gene are forcibly expressed, the forced expression may be halted, and then the apoptosis suppressor gene may be forcibly expressed, or the oncogene, the Polycomb gene, and the apoptosis suppressor gene may be forcibly expressed, or the oncogene and the Polycomb gene may be forcibly expressed and then the apoptosis suppressor gene may be expressed. As a result, it is possible to obtain the immortalized megakaryocytes in the megakaryocyte induction step. Further, in a later-described platelet production step, for example, by halting the forced expression of the immortalized megakaryocytes, the immortalized megakaryocytes become mature and the platelets are produced.

Because the megakaryocyte induction step allows improvement in the efficiency to introduce each gene, for example, the megakaryocyte induction step preferably includes a first expression step of forcibly expressing an oncogene and a Polycomb gene in cells that are less differentiated than the megakaryocytes, a second expression step of forcibly expressing an apoptosis suppressor gene such as Bcl-xL gene in the cells that are less differentiated than the megakaryocytes, and a halting step of halting all the forced expression. As described above, by halting the forced expression, for example, multinucleated megakaryocytes are induced from the immortalized megakaryocytes and the platelets are produced. Therefore, the halting step may also be referred to as a platelet production step, which will be described later.

Forced expression and the halting of forced expression of each gene can be carried out using a known method such as the methods described in WO 2011/034073, WO 2012/157586, WO 2014/123242 or Reference 1 below, for example, or a method that conforms with these methods. As a specific example, the forced expression and the halting of the forced expression of each gene can be, for example, carried out using a pharmaceutical agent responsive gene expression induction system. Examples of the gene expression induction system include TET-ON^{®} system, and TET-OFF^{®} system. If the TET-ON^{®} system is used, for example, in the forced expression step, culturing is carried out in the presence of a pharmaceutical agent for inducing gene expression, such as tetracycline and doxycycline, and in the halting step of forced expression, the culturing is carried out in the absence of the pharmaceutical agent. Reference 1: Nakamura S et al, "Expandable megakaryocyte cell lines enable clinically applicable generation of platelets from human induced pluripotent stem cells. ", Cell Stem Cell, 2014, vol. 14, No.4, pages 535-548

The "oncogene" in the present invention refers to a gene that is capable of inducing canceration of cells in an organism, and examples thereof include MYC family genes such as c-MYC, N-MYC, and L-MYC, and SRC family genes, RAS family genes, RAF family genes, c-kit (CD 117), PDGFRs (platelet-derived growth factor receptors), and protein kinase family genes such as Abl (Abelson murine leukemia viral oncogene homolog).

The "Polycomb gene" in the present invention refers to a gene that is known to function for negatively regulating CDKN2a (cyclin-dependent kinase inhibitor 2A, INK4a/ARF) and avoiding cellular aging (References 2 to 4 below). Specific examples of the Polycomb gene include BMI1 (Polycomb complex protein BMI-1, Polycomb group RING finger protein 4 (PCGF4), RING finger protein 51 (RNF51)), Mel18 (Polycomb group RING finger protein 2), Ring (Ring Finger Protein) 1a/b, Phc (Polyhomeotic Homolog) 1/2/3, Cbx (Chromobox) 2/4/6/7/8, Ezh2 (Enhancer of Zeste 2 Polycomb Repressive Complex 2 Subunit), Eed (Embryonic Ectoderm Development), Suz12 (SUZ12 Polycomb Repressive Complex 2 Subunit), HADC (Histone deacetylases), and Dnmt (DNA (cytosine-5)-methyltransferase) 1/3a/3b. Reference 2: Hideyuki Oguro et al., "Senescence and Ageing of Stem Cells Regulated by Polycomb Complexes", Regenerative Medicine, 2007, vol. 6, no. 4, pages 26 - 32. Reference 3: Jesus Gil et al., "Regulation of the INK4b-ARF-INK4a tumour suppressor locus: all for one or one for all", Nature Reviews Molecular Cell Biology, 2007, vol. 7, pages 667-677. Reference 4: Soo-Hyun Kim et al., "Absence of p16INK4a and truncation of ARF tumor suppressors in chickens", PNAS, 2003, vol. 100, No.1, pages 211-216.

The "apoptosis suppressor gene" in the present invention refers to a gene that functions to be capable of suppressing cell apoptosis, and examples thereof include BCL2 (B-cell lymphoma 2), Bcl-xL (B-cell lymphoma-extra large), Survivin (Baculoviral IAP Repeat Containing 5), and MCL1 (BCL2 Family Apoptosis Regulator).

The method for producing multinucleated megakaryocytes according to the present invention may include a proliferation step of proliferating the pre-multinucleated megakaryocyte cells in the presence of a growth factor of the pre-multinucleated megakaryocyte cells. In this case, in the proliferation step, the pre-multinucleated megakaryocyte cells are proliferated in the absence of harmine without suppressing the activities of AhR, ROCK, DYRK, and myosin 2. As a result, in the proliferation step, the pre-multinucleated megakaryocyte cells can be proliferated while the state of the pre-multinucleated megakaryocyte cells is maintained. Specifically, in the proliferation step, pre-multinucleated megakaryocytes are cultured in the presence of a growth factor of the pre-multinucleated megakaryocyte cells to proliferate the pre-multinucleated megakaryocytes. The growth factor of the pre-multinucleated megakaryocyte cells may be present in a culture solution or in the pre-multinucleated megakaryocytes. Also, in the proliferation step, when a substance that inhibits the activities of AhR, ROCK, DYRK, and myosin 2 and harmine are not added to the culture solution, the pre-multinucleated megakaryocytes can be proliferated in the absence of harmine without suppressing the activities of AhR, ROCK, DYRK, and myosin 2. To a culture medium, culture conditions, and the like used in the proliferation step, for example, the later-provided description of the multinucleation step can be applied.

Examples of growth factors of the pre-multinucleated megakaryocyte cells include stem cell factor (SCF) and thrombopoietin (TPO).

There is no particular limitation on the concentration of the growth factor of the pre-multinucleated megakaryocyte cells, and the concentration thereof can be set as appropriate according to, for example, the type and the effective concentration of the growth factor. When the growth factor is SCF or TPO, the concentration of the growth factor is in a range of, for example, 0.1 to 1000 ng/ml, or 1 to 200 ng/ml.

A culture period of the proliferation step can be, for example, set to a period taken to obtain a desired number of pre-multinucleated megakaryocytes. Specifically, the culture period of the proliferation step is 1 day or more, for example, and specific examples thereof include 1 day to 20 days, 1 day to 15 days, 1 day to 10 days, 1 day to 5 days, 2 days to 5 days, or 2 days to 4 days.

When the immortalized megakaryocytes are used as the pre-multinucleated megakaryocytes, if the oncogene and the Polycomb gene are expressed, or if the oncogene, the Polycomb gene, and the apoptosis suppressor gene are expressed, the immortalized megakaryocytes are proliferated while the state of the pre-multinucleated immortalized megakaryocytes is maintained. Thus, when the immortalized megakaryocytes are used, the first expression step and/or the second expression step can also be referred to as a proliferation step. Also, proteins encoded by the oncogene, the Polycomb gene, and/or the apoptosis suppressor gene, that is, proteins expressed from the oncogene, the Polycomb gene, and/or the apoptosis suppressor gene, can also be referred to as the growth factor of the pre-multinucleated megakaryocyte cells. When the immortalized megakaryocytes are used as the pre-multinucleated megakaryocytes, the description of the megakaryocyte induction step regarding the immortalized megakaryocytes can be applied to the proliferation step.

Then, in the method for producing multinucleated megakaryocytes according to the present invention, in the multinucleation step, multinucleated megakaryocytes are induced from the pre-multinucleated megakaryocyte cells in the presence of a growth factor of the pre-multinucleated megakaryocyte cells. Also, in the multinucleation step, multinucleated megakaryocyte cells are induced by suppressing the activity of at least one protein selected from the group consisting of AhR, ROCK, DYRK, and myosin 2, and multinucleating the pre-multinucleated megakaryocyte cells in the presence of harmine. Specifically, in the multinucleation step, the multinucleated megakaryocytes are induced by culturing the pre-multinucleated megakaryocytes in the presence of harmine, the growth factor of the pre-multinucleated megakaryocyte cells, and at least one substance selected from the group consisting of an AhR activity suppressing substance, a ROCK activity suppressing substance, a DYRK activity suppressing substance, and a myosin 2 activity suppressing substance. The growth factor of the pre-multinucleated megakaryocyte cells may be present in a culture solution or in the pre-multinucleated megakaryocytes. The AhR activity suppressing substance, the ROCK activity suppressing substance, the DYRK activity suppressing substance, the myosin 2 activity suppressing substance, and/or harmine are, for example, added to a culture medium for the pre-multinucleated megakaryocytes. Because expansion of megakaryocytes is induced in the multinucleation step, the multinucleation step can also be referred to, for example, as an expansion step. When a growth factor of the pre-multinucleated megakaryocyte cells, the AhR activity suppressing substance, the ROCK activity suppressing substance, the DYRK activity suppressing substance, and/or the myosin 2 activity suppressing substance are proteins or nucleic acid molecules, they may be introduced into the pre-multinucleated megakaryocytes through lipofection, electroporation, or the like in the multinucleation step.

In the multinucleation step, the activity of any one of AhR, ROCK, DYRK, and myosin 2, or the activities of two or more thereof may be suppressed. In the latter case, a combination of targets for suppressing activities may be any combination, and is preferably a combination of AhR and myosin 2, a combination of AhR, ROCK, and myosin 2, a combination of AhR, DYRK, and myosin 2, or a combination of AhR, ROCK, DYRK, and myosin 2 because these combinations realize high capability of facilitating multinucleation or improving platelet production. Therefore, preferably, the multinucleation step is carried out in the presence of harmine while suppressing the activities of AhR and myosin 2, the multinucleation step is carried out in the presence of harmine while suppressing the activities of AhR, ROCK, and myosin 2, the multinucleation step is carried out in the presence of harmine while suppressing the activities of AhR, DYRK, and myosin 2, or the multinucleation step is carried out in the presence of harmine while suppressing the activities of AhR, ROCK, DYRK, and myosin 2.

The AhR refers to aryl hydrocarbon receptor. The AhR is normally present in the cytoplasm, and upon activation, the AhR translocates into the nucleus and exhibits transcriptive activity.

In the multinucleation step, suppression of the AhR activity indicates that the AhR activity deteriorates as compared to that in the absence of the AhR activity suppressing substance. The AhR activity can be measured using, for example, translocation of the AhR into the nucleus as an indicator. The AhR activity can be measured using, for example, a commercially available AhR protein activity measurement kit, and a specific example thereof is a nuclear receptor assay kit (manufactured by PURACYP). In the present invention, the suppression of activity can also be referred to, for example, as inhibition of activity, deterioration in activity, suppression of activation, prevention of activation, and the like.

The AhR is a transcription factor belonging to the Per/ARNT/SIM (PAS) family. The AhR is inactive in a state in which no ligand is bound to the AhR, for example, and when an aromatic hydrocarbon compound binds thereto as a ligand, the AhR translocates into the nucleus. Then, after the nuclear translocation, the AhR forms a heterodimer with ARNT (Ahr Nuclear Translocator), for example, and binds to a xenobiotic responsive element (XRE, also referred to as "DRE"), thus activating transcription.

There is no particular limitation on the AhR inhibitor, and examples thereof include AhR antagonists, and expression suppressing nucleic acid molecules capable of suppressing AhR expression. There is no particular limitation on the AhR antagonists, and examples thereof include 4-(2-(2-(Benzo[b]thiophen-3-yl)-9-isopropyl-9H-purin-6-ylamino)ethyl)phenol (SR1), α-naphthoflavone (CAS 604-59-1), 1,4-dihydroxyanthraquinone, 1,5-dihydroxyanthraquinone, 1,8-dihydroxyanthraquinone, galangin (CAS 548-83-4), resveratrol, 2-methyl-2H-pyrazole-3-carboxylic acid (2-methyl-4-o-tolylazo-phenyl)-amide (CH-223191), N-(2-(3H-Indol-3-yl)ethyl)-9-isopropyl-2-(5-methyl-3-pyridyl)-7H-purin-6-amine (GNF351), 2-(29- amino-39-methoxyphenyl)-oxanaphthalen-4-one (PD98059), (Z)-3-[(2,4-dimethylpyrrol-5-yl)methylidenyl]-2-indolinone (TSU-16), 6, 2', 4'-trimethoxyflavone (TMF), and 3'-4'-dimethoxyflavone (DMF). Also, the AhR antagonist may be, for example, a compound described as an AhR antagonist in WO 2012/015914. Examples of the AhR expression suppressing nucleic acid molecules include expression suppressing nucleic acid molecules such as siRNA and miRNA targeting mRNA that encodes the AhR. An expression suppressing nucleic acid molecule for the AhR can be, for example, set as appropriate based on a base sequence of AhR mRNA registered in a database. A specific example of human AhR mRNA is, for example, a polynucleotide comprising a base sequence registered in Genbank with accession number: NM_001621. The AhR activity suppressing substances may be used alone or in combination of two or more.

In the multinucleation step, there is no particular limitation on the concentration of the AhR inhibitor, and the concentration thereof can be determined as appropriate according to the type and the effective concentration of a compound. Examples of the concentration of the AhR inhibitor include the following concentrations:
SR1: 200 nmmol/L or more and less than 1000 mmol/L,
CH-223191: 0.2 µmol/L or more and less than 4 µmol/L,
GNF351: 20 nmol/L or more and less than 300 nmol/L, and 20 nmol/L or more and less than 1000 nmol/L,
TMF: 2.5 µmol/L or more and less than 40 µmol/L, and
DMF: 2.5 µmol/L or more and less than 40 µmol/L.

The ROCK refers to Rho-associated coiled-coil forming kinase (ROCK).

In the multinucleation step, suppression of ROCK activity indicates, for example, that the ROCK activity deteriorates as compared to that in the absence of the ROCK activity suppressing substance. The ROCK activity can be measured, for example, using phosphorylation of a substrate by ROCK as an indicator. The ROCK activity can be measured using, for example, a commercially available ROCK protein activity measurement kit, and a specific example thereof is a ROCK activity measurement kit (96-Well ROCK Activity Assay Kit manufactured by Cell Bioloabs). In the present invention, the suppression of activity can also be referred to as, for example, inhibition of activity, deterioration in activity, suppression of activation, prevention of activation, and the like.

Examples of the ROCK inhibitor include ROCK antagonists, and expression suppressing nucleic acid molecules capable of suppressing ROCK expression. Examples of the ROCK inhibitor include (R)-(+)-trans-N-(4-pyridyl)-4-(1-aminoethyl)-cyclohexanecarboxamide (Y-27632), 4-[(1R)-1-aminoethyl]-N-(1H-pyrrolo[2,3-b]pyridin-4-yl)benzamide (Y-39983), Fasudil salt (Fasudil (HA1077) hydrochloride), 4-fluoro-5-[[(2S)-hexahydro-2-methyl-1H-1,4-diazepin-1-yl]sulfonyl]-isoquinoline (ripasudil), 2-(3-(4-((1H-indazol-5-yl)amino)quinazolin-2-yl)phenoxy)-N-isopropylacetamide (SLx-2119), N-[(3-Hydroxyphenyl)methyl]-N'-[4-(4-pyridinyl)-2-thiazolyl]urea dihydrochloride (RKI-1447), 6-chloro-N4-[3,5-difluoro-4-[(3-methyl-1H-pyrrolo[2,3-b]pyridin-4-yl)oxy]phenyl]-2,4-pyrimidinediamine (TC-S 7001, azaindole 1), N-[2-[2-(dimethylamino)ethoxy]-4-(1H-pyrazol-4-yl)phenyl-2,3-dihydro-1,4-benzodioxin-2-carboxamide (SR-3677), staurosporine, (S)-(+)-2-methyl-1-[(4-methyl-5-isoquinolinyl)sulfonyl]homopiperazine (H-1152), rac-(2R)-2-(dimethylamino)-N-(1-oxo- 1,2-dihydroisoquinolin-6-yl)-2-(thiophen-3-yl)acetamide (AR-12286), and N-(1-{[4-(methylsulfanyl)phenyl]methyl}piperidin-3-yl)-1H-indazol-5-amine (INS-117548). Examples of the ROCK expression suppressing nucleic acid molecules include expression suppressing nucleic acid molecules such as siRNA and miRNA targeting mRNA that encodes the ROCK. An expression suppressing nucleic acid molecule for the ROCK can be, for example, set as appropriate based on a base sequence of ROCK mRNA registered in a database. A specific example of human ROCK1 mRNA is a polynucleotide comprising a base sequence, for example, that registered in Genbank with accession number: NM_005406. Also, one example of human ROCK2 mRNA is a polynucleotide comprising a base sequence registered in Genbank with accession number: NM_004850. The ROCK activity suppressing substances may be used alone or in combination of two or more.

In the multinucleation step, there is no particular limitation on the concentration of the ROCK inhibitor, and the concentration thereof can be determined as appropriate according to the type and the effective concentration of a compound. When the ROCK activity suppressing substance is Y-39983, the concentration of the ROCK activity suppressing substance is 1×10⁻⁸ to 1×10⁻³ mol/l, for example, and preferably 1×10⁻⁷ to 1×10⁻⁵ mol/l.

The DYRK refers to dual-specificity tyrosine phosphorylation-regulated kinases.

In the suppression of the DYRK activity, examples of a target DYRK include DYRK1A, DYRK1B, and DYRK2, and DYRK1A is preferable. A case in which the DYRK1A activity is inhibited will be described below as an example.

In the multinucleation step, suppression of the DYRK1A activity indicates that the DYRK1A activity deteriorates as compared to that in the absence of the DYRK1A activity suppressing substance. The DYRK1A activity can be measured, for example, using a measurement system in which the DYRK1A protein and a substrate are used, and phosphorylation of the substrate is used as an indicator. As the former, for example, a commercially available DYRK1A protein activity measurement kit can be used, and a specific example thereof is DYRK1A Kinase Enzyme System (manufactured by Promega, Cat. No. VA7423, VA7424). As the latter, for example, Reference 5 below can be referred to. In the present invention, the suppression of activity can also be referred to as, for example, inhibition of activity, deterioration in activity, suppression of activation, prevention of activation, and the like. Reference 5: Isao Kii et al., "Selective inhibition of the kinase DYRK1A by targeting its folding process", Nat. Commun., 2016, vol. 7, article number 11391

There is no particular limitation on the DYRK1A activity suppressing substance, and examples thereof include compounds, proteins, and nucleic acid molecules that suppress the activity or expression of the DYRK1A, or the like. As the DYRK1A activity suppressing compound, for example, a known DYRK1A activity suppressing compound (DYRK1A inhibitor) can be used.. Specific examples of the DYRK1A activity suppressing compound (DYRK1A antagonists) include harmine (7-methoxy-1-methyl-9H-pyrido[3,4-b]indole, Cas No. 442-51-3), INDY((1Z)-1-(3-ethyl-5-hydroxy-2(3H)-benzothiazolylidene)-2-propanone, Pubchem No.: 69538603), GSK626616 ((5Z)-2-[(2,6-dichlorophenyl)amino]-5-(6-quinoxalinylmethylene)-4(5H)-thiazolone, Cas No. 1025821-33-3), TBB (casein kinase 2 inhibitor 1, 4,5,6,7-tetrabromobenzotriazole, Cas No. 17374-26-4), AZ191 (N-[2-methoxy-4-(4-methyl-1-piperazinyl)phenyl]-4-(1-methyl-1H-pyrrolo[2,3-c]pyridin-3-yl)-2-pyrimidinamine, Cas No. 1594092-37-1), Mrik-IN-1(TC-S 7004, N-[2-chloro-5-[[[(3-chlorophenyl)methyl]amino]carbonyl]phenyl]-7,8-dihydro-2-methoxy-7-oxopyrido[2,3-d]pyrimidine-6-carboxamide, Cas No. 1386979-55-0), EHT5372 (methyl 9-((2,4-dichlorophenyl)amino)thiazolo[5,4-f]quinazoline-2-carbimidate, Cas No. 1425945-60-3), GNF4877 ((R)-1-(3-(3-amino-6-(2-fluoro-5-isopropoxyphenyl)pyrazine-2-carboxamido)pyridin-4-yl)piperidine-3-carboxylic acid, Cas No. 2041073-22-5), FINDY ((5Z)-5-[[4-methoxy-3-[2-(trimethylsilyl)ethynyl]phenyl]methylene]-2-thioxo-4-thiazolidinone, Cas No. 1507367-37-4), ML351 (5-(methylamino)-2-(1-naphthalenyl)-4-oxazolecarbonitrile, Cas No. 847163-28-4), CLK-IN-T3 (N-(6-(3,5-di-tert-butylphenyl)imidazo[1,2-a]pyridin-2-yl)-4-(2-methyl-1-(4-methylpiperazin-1-yl)-1-oxopropan-2-yl)benzamide, Cas No. 2109805-56-1), casein kinase 2 inhibitor IX.IQA (5-oxo-5,6-dihydroindolo[1,2-a]quinazoline-7-acetic acid, Cas No. 391670-48-7), protein kinase inhibitor 1 ((E)-5-((2-oxo-6'-(piperazin-1-yl)-1,2-dihydro-[3,3'-bipyridin]-5-yl)methylene)thiazolidine-2,4-dione hydrochloride, Cas No. 1365986-44-2), and leucettine L41 ((SZ)-5-(1,3-benzodioxol-5-ylmethylene)-3,5-dihydro-2-(phenylamino)-4H-imidazol-4-one, Cas No. 1112978-84-3). The DYRK1A activity suppressing compound, for example, does not need harmine. As described above, the multinucleation step is carried out in the presence of harmine. Therefore, it is preferable to use compounds other than the harmine as the DYRK1A activity suppressing compound in addition to harmine, and a specific example thereof is AZ191. Examples of the DYRK1A activity suppressing protein include dominant negative form (DN) protein of DYRK1A. Specific examples of the human DYRK1ADN protein include mutant proteins in which lysine (K) at position 188 is replaced with arginine (R). Examples of the DYRK1A expression suppressing nucleic acid molecules include expression suppressing nucleic acid molecules such as siRNA and miRNA targeting mRNA that encode the DYRK1A. An expression suppressing nucleic acid molecule for the DYRK1A can be, for example, set as appropriate based on a base sequence of DYRK1A mRNA registered in a database. A specific example of human DYRK1A mRNA is a polynucleotide comprising a base sequence registered in Genbank with accession number: NM_001396, NM_101395, NM_130436, NM_130437, NM_130438, NM_001347721, NM_001347722, or NM_001347723. The DYRK1A activity suppressing substances may be, for example, used alone or in combination of two or more.

There is no particular limitation on the concentration of the DYRK1A activity suppressing substance, and the concentration thereof can be set as appropriate according to the type and the effective concentration of the DYRK1A activity suppressing substance. When the DYRK1A activity suppressing substance is harmine, for example, the concentration of the DYRK1A activity suppressing substance is 1×10⁻⁸ to 1×10⁻³ mol/l, for example, and preferably 1×10⁻⁶ to 1×10⁻⁴ mol/l.When the DYRK1A activity suppressing substance is AZ191, the concentration of the DYRK1A activity suppressing substance is 1×10⁻⁹ to 1×10⁻³ mol/l, for example, and preferably 1×10⁻⁷ to 1×10⁻⁵ mol/l.

The myosin 2 is a protein that has ATPase activity and moves on actin, and refers to a complex containing two heavy chains and two light chains (four chains in total), which are respectively attached to the heavy chains.

In the multinucleation step, suppression of myosin 2 activity indicates that the ATPase activity of myosin 2 deteriorates as compared to that in the absence of the myosin 2 activity suppressing substance. The myosin 2 activity can be, for example, measured using phosphoric acid released from myosin 2 as an indicator. The myosin 2 activity can be for example, measured using a commercially available myosin 2 protein activity measurement kit. In the present invention, for example, the suppression of activity can also be referred to as inhibition of activity, deterioration in activity, suppression of activation, prevention of activation, and the like.

Examples of the myosin 2 inhibitor include myosin 2 antagonists, expression suppressing nucleic acid molecules capable of suppressing the expression of myosin 2, and myosin light-chain kinase (MLCK) inhibitors. Examples of the myosin 2 inhibitors include 3a-hydroxy-6-methyl-1-phenyl-2,3-dihydropyrrolo[2,3-b]quinolin-4-one (Blebbistatin), CK1122534, omecamtiv mecarbil (methyl 4-[[2-fluoro-3-[(6-methylpyridin-3-yl)carbamoylamino]phenyl]methyl]piperazine-1-carboxylate), ammosamides A&B, CTK2018448, and manassantin B ((1R,2R)-1-(1,3-benzodioxol-5-yl)-2-[4-[(2S,3R,4R,SS)-5-[4-[(1R,2R)-1-(3,4-dimethoxyphenyl)-1-hydroxypropan-2-yl]oxy-3-methoxyphenyl]-3,4-dimethyloxolan-2-yl]-2-methoxyphenoxy]propan-1-ol). Examples of the MLCK inhibitors include ML7 (hexahydro-1-[(5-iodo-1-naphthalenyl)sulfonyl]-1H-1,4-diazepine) and ML9 (1-(5-chloronaphthalene-1-sulfonyl)-1H-hexahydro-1,4-diazepine). Examples of the myosin 2 expression suppressing nucleic acid molecules include expression suppressing nucleic acid molecules such as siRNA and miRNA targeting mRNA that encode the myosin 2 or myosin light-chain kinase. An expression suppressing nucleic acid molecule for the myosin 2 can be, for example, set as appropriate based on a base sequence of mRNA of heavy chains or light chains that constitute myosin 2, or myosin light-chain kinase registered in a database. A specific example of mRNA of the heavy chains included in human myosin 2 is a polynucleotide comprising a base sequence registered in Genbank with accession number: NM_005963, NM_001100112, NM_017534, NM_002470, NM_017533, NM_002147, NM_002471, NM_000257, NM_002472, NM_002473, NM_001256012, NM_002474, NM_001382347, NM_003802, NM_001145809, or NM_014981. An example of mRNA of the light chain included in human myosin 2 is a polynucleotide comprising a base sequence registered in Genbank with accession number: NM_079420, NM_000432, NM_000258, NM_002476, NM_001363650, NM_021019, NM_021223, NM_006097, NM_138403, or NM_001324458. An example of human myosin light-chain kinase mRNA is a polynucleotide comprising a base sequence registered in Genbank with accession number: NM_001321309, NM_053025, NM_053026, NM_053027, NM_053028, NM_053031, NM_053032, NM_033118, NM_053032, or NM_18249. The myosin 2 activity suppressing substances may be used alone or in combination of two or more.

In the multinucleation step, there is no particular limitation on the concentration of the myosin 2 inhibitor, and the concentration thereof can be determined as appropriate according to the type and the effective concentration of a compound. When the myosin 2 activity suppressing substance is blebbistatin, the concentration of the myosin 2 activity suppressing substance is 1×10⁻⁸ to 1×10⁻³ mol/l, for example, and preferably 1×10⁻⁶ to 1×10⁻⁴ mol/l.

In the multinucleation step, the concentration of the harmine is, for example, 1×10⁻⁸ to 1×10⁻³ mol/l, and preferably 1×10⁻⁶ to 1×10⁻⁴ mol/l.

In the multinucleation step, suppression of the activity of AhR, ROCK, DYRK and/or myosin 2 and coexistence of AhR, ROCK, DYRK and/or myosin 2 with harmine may be realized at the same time or at different times, but are preferably realized at the same time. That is, it is preferable that the multinucleated megakaryocytes are induced from the pre-multinucleated megakaryocytes in a state in which the activity of at least one protein selected from the group consisting of AhR, ROCK, DYRK, and myosin 2 is suppressed and harmine coexists therewith.

When the pre-multinucleated megakaryocytes are immortalized megakaryocytes, for example, the multinucleation step can be carried out as follows. Specifically, as described above, proteins expressed from the oncogene, the Polycomb gene, and/or the apoptosis suppressor gene function as growth factors of pre-multinucleated megakaryocyte cells in the immortalized megakaryocytes. In view of this, when the immortalized megakaryocytes are used, multinucleated megakaryocytes are induced by maintaining the forced expression of the oncogene, the Polycomb gene, and/or the apoptosis suppressor gene, suppressing the activity of at least one protein selected from the group consisting of AhR, ROCK, DYRK, and myosin 2, and culturing the immortalized megakaryocytes in the presence of harmine. In this case, in the multinucleation step, multinucleated megakaryocytes can be induced by, while the immortalized megakaryocytes are being cultured, replacing a culture medium containing the immortalized megakaryocytes with a culture medium containing harmine and at least one inhibitor selected from the group consisting of the AhR inhibitor, the ROCK inhibitor, the DYRK inhibitor, and the myosin 2 inhibitor, or by adding, to a culture medium containing the immortalized megakaryocytes, harmine and at least one inhibitor selected from the group consisting of the AhR inhibitor, the ROCK inhibitor, the DYRK inhibitor, and the myosin 2 inhibitor. The addition or replacement above may be for example, carried out once, or twice or more times.

There is no particular limitation on a culture medium used in the multinucleation step, for example, and examples thereof include known culture media that are suitable for producing the megakaryocytes or platelets from megakaryocytes, and culture media that are equivalent thereto. As a specific example, the culture medium can be prepared using, for example, as a basal culture medium, a culture medium that is to be used to culture mammalian cells. Examples of the basal medium include single media such as IMDM, Medium 199, Eagle's Minimum Essential Medium (EMEM), αMEM, Dulbecco's modified Eagle's Medium (DMEM), Ham's F12 medium, RPMI1640 medium, Fischer's culture medium, and NEUROBASAL^{®}medium (manufactured by Thermo Fisher Scientific), and a mixed medium thereof. The culture medium may contain serum or blood plasma, for example, or may be a serum-free medium that does not contain serum or blood plasma. A serum-free medium is preferable. When a serum-free medium is used as a culture medium in the multinucleation step, for example, the degree of multinucleation of the pre-multinucleated megakaryocytes obtained from the pre-multinucleated megakaryocytes can be improved using the method for producing multinucleated megakaryocytes according to the present invention. Examples of the serum-free medium include StemSpan ACF (manufactured by StemCell Technologies, Cat. No.:9855) and Stemline 2 (manufactured by Sigma Aldrich, Cat. No. :S0192). The origins of the serum and the blood plasma are preferably the same as the origin of the megakaryocytes. When the megakaryocytes are derived from a human as a specific example, the serum and the blood plasma are each preferably derived from a human. When the origins of the megakaryocytes and the serum are the same, for example, multinucleation of the megakaryocytes can be further facilitated in the multinucleation step using the production method according to the present invention.

The culture medium may, for example, contain other components. There is no particular limitation on the other components, and examples thereof include albumin, insulin, transferrin, selenium, fatty acids, trace elements, 2-mercaptoethanol, thiolglycerol, monothioglycerol (MTG), lipids, amino acids (e.g., L-glutamine), ascorbic acid, heparin, non-essential amino acids, vitamins, growth factors, low molecular weight compounds, antibiotics, antioxidants, pyruvic acid, buffers, inorganic salts, and cytokines. The number of types of other components may be, for example, one, or two or more. The cytokine is, for example, a substance that promotes differentiation of hematopoietic cells, and specific examples thereof include vascular endothelial cell growth factor (VEGF), thrombopoietin (TPO), various TPO-like active substances, Stem Cell Factor (SCF), ITS (Insulin-Transferrin-Selenite) supplements, ADAM inhibitors, FLT inhibitors, and WNT inhibitors. The culture medium is, for example, preferably IMDM containing serum, insulin, transferrin, serine, thiolglycerol, ascorbic acid, and TPO. The culture medium may further contain, for example, SCF and heparin. There is no particular limitation on the concentration of the other components. The TPO concentration ranges, for example, from about 10 ng/ml to about 200 ng/ml, or from about 50 ng/ml to about 100 ng/ml. The SCF concentration ranges, for example, from about 10 ng/ml to about 200 ng/ml, or is about 50 ng/ml. The heparin concentration ranges, for example, from about 10 U/ml to about 100 U/ml, or is about 25 U/ml. The culture medium may further contain, for example, a phorbol ester (e.g., phorbol-12-myristate-13-acetate; PMA).

In the multinucleation step, there is no particular limitation on the initial cell density of pre-multinucleated megakaryocytes. The lower limit of the cell density, for example, is 1×10⁵ cells/ml, 2×10⁵ cells/ml, 3×10⁵ cells/ml, or 4×10⁵ cells/ml. There is no particular limitation on the upper limit of the cell density, and examples thereof are 4×10⁵ cells/ml, 6×10⁵ cells/ml, and 8×10⁵ cells/ml. The cell density ranges from, for example, 1×10⁵ cells/ml to 8×10⁵ cells/ml, from 1×10⁵ cells/ml to 8×10⁵ cells/ml, from 1×10⁵ cells/ml to 5×10⁵ cells/ml, from 2×10⁵ cells/ml to 8×10⁵ cells/ml, from 3×10⁵ cells/ml to 6×10⁵ cells/ml, or from 4×10⁵ cells/ml to 6×10⁵ cells/ml. Because multinucleation can be facilitated or production of platelets can be improved, the cell density preferably ranges from 1×10⁵ cells/ml to 8×10⁵ cells/ml, or 1×10⁵ cells/ml to 5×10⁵ cells/ml. The cell density can be, for example, calculated by dividing the number of the pre-multinucleated megakaryocytes by the volume of a culture medium in which the pre-multinucleated megakaryocytes are suspended.

There is no particular limitation on the culture conditions in the multinucleation step, and normal culture conditions for the megakaryocytes can be adopted. As specific examples, the culture temperature is in a range of about 35°C to about 42°C, about 36°C to about 40°C, or about 37°C to about 39°C. The CO₂ concentration is, for example, in a range of about 5% to about 15%. The O₂ concentration is, for example, in a range of about 15% to about 25%, and is about 20%.

The culture period in the multinucleation step can be, for example, set as a period taken until the pre-multinucleation megakaryocytes are multinucleated. The culture period in the multinucleation step may be set as appropriate according to a percentage of the multinucleated megakaryocytes in the megakaryocytes, which are being cultured. The culture period in the multinucleation step is preferably set to a period in which the percentage of multinucleated megakaryocytes having a nuclear phase of 8N or more in the megakaryocytes, which are being cultured, is 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 35% or more, 40% or more, 45% or more, 50% or more, 55% or more, or 60% or more. As a specific example, the culture period in the multinucleation step is 0 to 20 days, or 1 to 15 days. The percentage of multinucleated megakaryocytes in the megakaryocytes can be, for example, calculated by extracting a part of megakaryocytes that are being cultured, according to Example 1(3) described later.

In the multinucleation step, the pre-multinucleated megakaryocytes may be, for example, cultured on feeder cells, or may be cultured without feeder cells. The pre-multinucleated megakaryocytes can be cultured in suspension, for example, and thus the pre-multinucleated megakaryocytes can be cultured without the feeder cells. The feeder cells refer to, for example, cells that are co-cultured with cells (target cells) to be proliferated or differentiated, in order to prepare an environment necessary for culturing the target cells. The feeder cells may be cells derived from the same species as the target cells, or may be cells derived from different species, as long as the feeder cells can be distinguished from the target cells. The feeder cells may be, for example, cells that have been treated with antibiotics, anticancer drugs, γ-ray irradiation, or the like such that the treated feeder cells do not proliferate.

With the production method according to the present invention, after the multinucleation step, multinucleated megakaryocytes are present. Multinucleated megakaryocytes obtained after the multinucleation step may be, for example, constituted by only multinucleated megakaryocytes or may contain other cells. In the latter case, the multinucleated megakaryocytes obtained after the multinucleation step can also be, for example, referred to as a cell population containing multinucleated megakaryocytes. The multinucleated megakaryocytes may be, for example, multinucleated megakaryocytes having a nuclear phase of 8N or more, multinucleated megakaryocytes having a nuclear phase of 16N or more, or multinucleated megakaryocytes having a nuclear phase of 32N or more. The multinucleated megakaryocytes obtained after the multinucleation step include, for example, the multinucleated megakaryocytes having a nuclear phase of 8N or more, the multinucleated megakaryocytes having a nuclear phase of 16N or more, and/or the multinucleated megakaryocytes having a nuclear phase of 32N or more. With the production method according to the present invention, for example, it is possible to increase the percentage of the multinucleated megakaryocytes having a nuclear phase of 16N or more and the multinucleated megakaryocytes having a nuclear phase of 32N or more.

When the multinucleated megakaryocytes include multinucleated megakaryocytes having a nuclear phase of 8N or more, the lower limit of the percentage of the multinucleated megakaryocytes having a nuclear phase of 8N or more (the number of cells) in the cell population is, for example, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 35% or more, 40% or more, 45% or more, 50% or more, 55% or more, or 60% or more. The upper limit of the percentage of the multinucleated megakaryocytes having a nuclear phase of 8N or more (the number of cells) in the cell population is, for example, 90% or less, 85% or less, 80% or less, 75% or less, 70% or less, 65% or less, 60% or less, 59% or less, 58% or less, 57% or less, 56% or less, 55% or less, 54% or less, 53% or less, 52% or less, 51% or less, 50% or less, 49% or less, 48% or less, 47% or less, or 46% or less. The percentage of the multinucleated megakaryocytes having a nuclear phase of 8N or more (the number of cells) is in a range of, for example, 15% to 90%, 20% to 90%, 25% to 90%, 30% to 90%, 35% to 90%, 40% to 90%, 45% to 90%, 50% to 90%, 55% to 90%, 60% to 90%, 15% to 85%, 15% to 80%, 15% to 75%, 15% to 70%, 15% to 65%, 16% to 60%, 17% to 59%, 18% to 58%, 19% to 57%, 20% to 56%, 21% to 55%, 22% to 54%, 23% to 54%, 24% to 53%, 25% to 51%, 26% to 50%, 27% to 49%, 28% to 48%, 29% to 47%, or 30% to 46%. The percentage (the number of cells) can be measured using, for example, a method in which a target cell population is sufficiently suspended, a part of the obtained cell suspension is fractionated, and the number of sets of chromosomes (nuclear phase) is measured according to Example 1(3), which will be described later (the same applies to the following).

When the multinucleated megakaryocytes include multinucleated megakaryocytes having a nuclear phase of 16N or more, the lower limit of the percentage of the multinucleated megakaryocytes having a nuclear phase of 16N or more (the number of cells) in the cell population is, for example, 15% or more, 16% or more, 17% or more, 18% or more, 19% or more, 20% or more, 21% or more, 22% or more, 23% or more, 24% or more, or 25% or more. The upper limit of the percentage of the multinucleated megakaryocytes having a nuclear phase of 16N or more (the number of cells) in the cell population is, for example, 90% or less, 85% or less, 80% or less, 75% or less, 70% or less, 65% or less, 60% or less, 55% or less, 54% or less, 53% or less, 52% or less, 51% or less, 50% or less, 49% or less, 48% or less, 47% or less, 46% or less, 45% or less, 44% or less, or 43% or less. The percentage of the multinucleated megakaryocytes having a nuclear phase of 16N or more (the number of cells) is in a range of, for example, 15% to 90%, 15% to 85%, 15% to 80%, 15% to 75%, 15% to 70%, 15% to 65%, 15% to 60%, 16% to 55%, 17% to 54%, 18% to 53%, 19% to 52%, 20% to 51%, 21% to 50%, 22% to 49%, 23% to 48%, 24% to 47%, 25% to 46%, 25% to 45%, 25% to 44%, or 25% to 43%.

When the multinucleated megakaryocytes include multinucleated megakaryocytes having a nuclear phase of 32N or more, the lower limit of the percentage of the multinucleated megakaryocytes having a nuclear phase of 32N or more (the number of cells) in the cell population is, for example, 5% or more, 6% or more, 7% or more, 8% or more, 9% or more, 10% or more, 11% or more, 12% or more, 13% or more, 14% or more, or 15% or more. The upper limit of the percentage of the multinucleated megakaryocytes having a nuclear phase of 32N or more (the number of cells) in the cell population is, for example, 50% or less, 49% or less, 48% or less, 47% or less, 46% or less, 45% or less, 44% or less, 43% or less, 42% or less, 41% or less, 40% or less, 39% or less, 38% or less, 37% or less, 36% or less, 35% or less, 34% or less, 33% or less, 32% or less, 31% or less, or 30% or less. The percentage of the multinucleated megakaryocytes having a nuclear phase of 32N or more (the number of cells) is in a range of, for example, 5% to 50%, 5% to 49%, 5% to 48%, 5% to 47%, 5% to 46%, 5% to 45%, 5% to 44%, 5% to 43%, 5% to 42%, 5% to 41%, 5% to 40%, 6% to 39%, 7% to 38%, 8% to 37%, 9% to 36%, 10% to 35%, 11% to 34%, 12% to 33%, 13% to 32%, 14% to 31%, 15% to 31%, or 15% to 30%.

Megakaryocytes with enhanced platelet production capability can be produced using the method for producing multinucleated megakaryocytes according to the present invention in this manner. With the method for producing multinucleated megakaryocytes according to the present invention, it is possible to obtain multinucleated megakaryocytes with an increased nuclear phase, in particular, a cell population in which multinucleated megakaryocytes having a nuclear phase of 16N and 32N are enriched. Therefore, it is possible to obtain multinucleated megakaryocytes with enhanced platelet production capability, using the method for producing multinucleated megakaryocytes according to the present invention. Thus, the multinucleated megakaryocytes have enhanced platelet production capability. Therefore, multinucleated megakaryocytes capable of improving the amount of platelets produced can be prepared using the method for producing multinucleated megakaryocytes according to the present invention.

### Cell population containing multinucleated megakaryocytes

In another embodiment, the present invention provides a cell population containing multinucleated megakaryocytes with enhanced platelet production capability. A cell population according to the present invention is a cell population containing multinucleated megakaryocytes, and the multinucleated megakaryocytes include multinucleated megakaryocytes having a nuclear phase of 16N or more, and the percentage of the multinucleated megakaryocytes having a nuclear phase of 16N or more (the number of cells) in the cell population is 15% or more.

Also, the cell population according to the present invention is a cell population containing multinucleated megakaryocytes, and the multinucleated megakaryocytes include multinucleated megakaryocytes having a nuclear phase of 32N or more, and the percentage of the multinucleated megakaryocytes having a nuclear phase of 32N or more (the number of cells) in the cell population is in a range of 5% to 50%.

The multinucleated megakaryocytes may include multinucleated megakaryocytes having a nuclear phase of 8N or more. The above-described examples can be, for example, applied to the percentage of the multinucleated megakaryocytes having a nuclear phase of 8N or more, the multinucleated megakaryocytes having a nuclear phase of 16N or more, and the multinucleated megakaryocytes having a nuclear phase of 32N or more.

The cell population according to the present invention contains a certain amount of multinucleated megakaryocytes having a predetermined nuclear phase, and there is no particular limitation on other constituents or conditions. A description of the method for producing multinucleated megakaryocytes according to the present invention can be, for example, applied to the cell population according to the present invention. Multinucleated megakaryocytes with a high nuclear phase are enriched in the cell population of the present invention. As nuclear phases of the megakaryocytes become multinucleated, the cytoplasm of the megakaryocytes becomes comparatively larger, thus improving platelet production capability. Also, the more nuclear phases of the megakaryocytes become multinucleated, the further the maturation of the megakaryocytes progresses. The percentage of multinucleated megakaryocytes with a particularly high nuclear phase (e.g., 16N or more or 32N or more) in the cell population according to the present invention is increased. Therefore, the cell population according to the present invention, for example, has enhanced platelet production capability.

The pre-multinucleated megakaryocyte cells are derived from pluripotent cells, for example, and preferably induced from pluripotent cells *in vitro.* The pre-multinucleated megakaryocytes are preferably immortalized megakaryocytes. As described above, the immortalized megakaryocytes can be, for example, prepared by introducing the oncogene, the Polycomb gene, and/or the apoptosis suppressor gene. Thus, the pre-multinucleated megakaryocytes and the multinucleated megakaryocytes, for example, contain an exogenous oncogene, Polycomb gene, and/or apoptosis suppressor gene. The oncogene is preferably c-MYC gene. The Polycomb gene is preferably BMI1 gene. The apoptosis suppressor gene is preferably BCL-xL gene. An exogenous oncogene, Polycomb gene, and/or apoptosis suppressor gene is expressed in the pre-multinucleated megakaryocytes and the multinucleated megakaryocytes.

The cell population according to the present invention can be, for example, produced using the method for producing multinucleated megakaryocytes according to the present invention. Also, by fractionating a cell population containing multinucleated megakaryocytes according to Example 1(5) described later using a serum albumin solution, for example, the cell population according to the present invention may be prepared as a cell population in which multinucleated megakaryocytes are enriched.

### Method for producing platelets

In another embodiment, the present invention provides a method for producing platelets. As described above, a method for producing platelets according to the present invention includes an induction step of inducing multinucleated megakaryocyte cells from pre-multinucleated megakaryocyte cells and a platelet production step of producing platelets from the multinucleated megakaryocyte cells, and the induction step is carried out using the method for producing multinucleated megakaryocytes according to the present invention. The method for producing platelets according to the present invention is characterized by the induction step being carried out using the method for producing multinucleated megakaryocytes according to the present invention, and there is no particular limitation on other steps and conditions. A description of the method for producing multinucleated megakaryocytes according to the present invention can be, for example, applied to the method for producing platelets according to the present invention. With the method for producing platelets according to the present invention, the amount of produced platelets is improved.

In the method for producing platelets according to the present invention, multinucleated megakaryocyte cells are induced from pre-multinucleated megakaryocyte cells in the induction step. The induction step can be carried out in a manner similar to that in the multinucleation step in the method for producing multinucleated megakaryocytes according to the present invention.

Then, as described above, platelets are produced from the multinucleated megakaryocytes in the platelet production step (also referred to as a "production step" hereinafter) in the method for producing platelets according to the present invention. The production step can be, for example, carried out by culturing the multinucleated megakaryocytes in a culture medium. The multinucleated megakaryocytes may be cultured on feeder cells, or may be cultured without feeder cells.

In the production step, there is no particular limitation on a period in which platelets are produced from the multinucleated megakaryocytes, and the period, for example, is in a range of 1 to 10 days, or 3 to 6 days.

The production step may be, for example, carried out in the presence of an AhR inhibitor because the physiological activity of produced platelets can be improved. Specifically, the production step can be, for example, carried out in a culture medium containing an AhR inhibitor. The production step can be, for example, carried out by replacing a culture medium containing the megakaryocytes with a culture medium containing an AhR inhibitor, for example, or adding an AhR inhibitor to a culture medium containing the megakaryocytes.

There is no particular limitation on a period in which megakaryocytes are cultured in the presence of the AhR inhibitor, and the period can be, for example, determined as appropriate according to the number, physiological activity, and the like of produced platelets. The AhR inhibitor is present during all or part of the production step. When the AhR inhibitor is SR1, the culture period is preferably about 5 days because highly functional platelets in particular can be obtained.

In the production step, there is no particular limitation on the concentration of the AhR inhibitor, and the concentration of the AhR inhibitor can be determined as appropriate according to the type and effective concentration of the compound. Examples of the concentration of the AhR antagonist are as follows because, for example, the physiological activity of platelets produced can be further improved.
SR1: 200 nmmol/L or more and less than 1000 mmol/L,
CH-223191: 0.2 µmol/L or more and less than 4 µmol/L,
GNF351: 20 nmol/L or more and less than 300 nmol/L,
TMF: 2.5 µmol/L or more and less than 40 µmol/L, and
DMF: 2.5 µmol/L or more and less than 40 µmol/L.

The production step may be, for example, carried out in the presence of a ROCK inhibitor because the physiological activity of produced platelets can be improved. Specifically, the production step can be, for example, carried out in a culture medium containing a ROCK inhibitor. It is preferable to use the ROCK inhibitor and the AhR inhibitor, for example, in combination in the production step because highly functional platelets can be obtained. The production step can be, for example, carried out by replacing a culture medium containing the megakaryocytes with a culture medium containing a ROCK inhibitor, for example, or adding a ROCK inhibitor to a culture medium containing the megakaryocytes. When the ROCK inhibitor and the AhR inhibitor are used, for example, in combination, the ROCK inhibitor and the AhR inhibitor may be added or replaced at the same time, or may be added or replaced separately.

In the production step, there is no particular limitation on the cell density of multinucleated megakaryocytes when the production of platelets is started. The lower limit of the cell density is, for example, 1×10⁵ cells/ml, 2×10⁵ cells/ml, 3×10⁵ cells/ml, or 4×10⁵ cells/ml. There is no particular limitation on the upper limit of the cell density, and examples thereof are4×10⁵ cells/ml, 6×10⁵ cells/ml, and 8×10⁵ cells/ml. The cell density ranges from, for example, 1×10⁵ cells/ml to 8×10⁵ cells/ml, from 2×10⁵ cells/ml to 8×10⁵ cells/ml, from 3×10⁵ cells/ml to 6×10⁵ cells/ml, or from 4×10⁵ cells/ml to 6×10⁵ cells/ml. The cell density can be calculated by dividing the number of the multinucleated megakaryocyte cells by the volume of a culture medium in which the multinucleated megakaryocyte cells are suspended.

There is no particular limitation on the culture conditions for the megakaryocytes in the production step, and normal culture conditions for the megakaryocytes can be adopted. As specific examples, the culture temperature is in a range of about 35°C to about 42°C, about 36°C to about 40°C, or about 37°C to about 39°C. The CO₂ concentration is, for example, in a range of about 5% to about 15%. The O₂ concentration is, for example, in a range of about 15% to about 25%, and is about 20%.

It is possible to produce platelets from the megakaryocytes in this manner. The culture medium obtained after the production step, for example, contains the platelets.

The method for producing platelets according to the present invention may include, for example, a purification step of purifying the platelets obtained in the production step. There is no particular limitation on the method for purifying platelets, and the platelets can be, for example, purified using a known method such as a purification method using a separation means such as a hollow fiber membrane, or a purification method through centrifugation.

### Platelets

Platelets according to the present invention are characterized by being obtained using the method for producing platelets according to the present invention. The platelets according to the present invention are obtained using the method for producing platelets according to the present invention, and there is no particular limitation on other steps and conditions. A description of the method for producing platelets according to the present invention can be, for example, applied to the platelet according to the present invention.

### Method for producing platelet preparation

As described above, a method for producing a platelet preparation according to the present invention includes a preparation step of producing a platelet preparation from platelets, and the platelets are obtained using the method for producing platelets according to the present invention. The method for producing a platelet preparation according to the present invention is characterized by the platelets being obtained using the method for producing platelets according to the present invention, and there is no particular limitation on other steps and conditions. A description of the method for producing platelets according to the present invention can be applied to the method for producing a platelet preparation according to the present invention.

Other components may be, for example, added in the preparation step. Examples of the other components include pharmaceutically acceptable additive agents such as stabilizers for cells such as the platelets, or pharmaceutically acceptable carriers.

The method for producing a platelet preparation according to the present invention may include a platelet production step of producing platelets using the method for producing platelets according to the present invention, prior to the preparation step. A description of the method for producing platelets according to the present invention can be, for example, applied to the platelet production step.

### Platelet preparation

A platelet preparation according to the present invention is characterized by being obtained using the method for producing a platelet preparation according to the present invention. The platelet preparation according to the present invention is obtained using the method for producing a platelet preparation according to the present invention, and there is no particular limitation on other steps and conditions. A description of the method for producing platelets according to the present invention and the method for producing a platelet preparation according to the present invention can be, for example, applied to the platelet preparation according to the present invention.

### Method for producing blood preparation

As described above, a method for producing a blood preparation according to the present invention includes a blood preparation step of producing a blood preparation by mixing platelets and other components together, and the platelets are obtained using the method for producing platelets according to the present invention. The method for producing a blood preparation according to the present invention is characterized by the platelets being obtained using the method for producing platelets according to the present invention, and there is no particular limitation on other steps and conditions. A description of the method for producing platelets according to the present invention can be applied to the method for producing a blood preparation according to the present invention.

There is no particular limitation on the other components, and examples of the other components include cells such as other hematopoietic cells (e.g., red blood cells); and additive agents such as stabilizers for cells such as the platelets.

The method for producing a blood preparation according to the present invention may include a platelet production step of producing platelets using the method for producing platelets according to the present invention, prior to the blood preparation step. A description of the method for producing platelets according to the present invention can be, for example, applied to the platelet production step.

### Blood preparation

A blood preparation according to the present invention is characterized by being obtained using the method for producing a blood preparation according to the present invention. The blood preparation according to the present invention is obtained using the method for producing a blood preparation according to the present invention, and there is no particular limitation on other steps and conditions. A description of the method for producing platelets according to the present invention and the method for producing a blood preparation according to the present invention can be applied to the blood preparation according to the present invention.

### Inducing agent for multinucleated megakaryocytes

In another embodiment, the present invention provides an inducing agent for inducing multinucleated megakaryocytes with enhanced platelet production capability. An inducing agent according to the present invention is an inducing agent for inducing multinucleated megakaryocytes with enhanced platelet production capability, and contains harmine and at least one substance selected from the group consisting of an AhR activity suppressing substance, a ROCK activity suppressing substance, a DYRK activity suppressing substance, and a myosin 2 activity suppressing substance. The inducing agent according to the present invention is characterized by containing harmine and at least one substance selected from the group consisting of an AhR activity suppressing substance, a ROCK activity suppressing substance, a DYRK activity suppressing substance, and a myosin 2 activity suppressing substance, and there is no particular limitation on other constituents and conditions. A description of the method for producing multinucleated megakaryocytes according to the present invention can be, for example, applied to the inducing agent according to the present invention. The inducing agent according to the present invention can also be, for example, referred to as an inducing agent for inducing mature megakaryocytes or multinucleated megakaryocyte cells with enhanced platelet production capability. Also, the inducing agent according to the present invention can also be, for example, referred to as an agent for accelerating multinucleation of megakaryocyte cells or an agent for increasing the nuclear phase of megakaryocyte cells.

### Use

The present invention also relates to use of harmine and at least one substance selected from the group consisting of an AhR activity suppressing substance, a ROCK activity suppressing substance, a DYRK activity suppressing substance, and a myosin 2 activity suppressing substance, for inducing multinucleated megakaryocytes with enhanced platelet production capability. Also, the present invention also relates to use of harmine and at least one substance selected from the group consisting of an AhR activity suppressing substance, a ROCK activity suppressing substance, a DYRK activity suppressing substance, and a myosin 2 activity suppressing substance for use to accelerate multinucleation of megakaryocyte cells or increase the nuclear phase of megakaryocytes. A description of the method for producing multinucleated megakaryocyte cells according to the present invention can be, for example, applied to the present invention.

### Examples

The following describes the present invention in detail using examples, but the present invention is not limited to aspects described in the examples.

### Example 1

It was confirmed that platelet production capability can be enhanced by culturing pre-multinucleated megakaryocytes in the presence of harmine, with an AhR inhibitor, a ROCK inhibitor, a DYRK inhibitor, and a myosin 2 inhibitor.

### (1) Production of immortalized megakaryocytes

Immortalized megakaryocytes were used as pre-multinucleated megakaryocytes. The immortalized megakaryocytes were produced using the following procedure.

### (1-1) Preparation of hematopoietic progenitor cells from iPS cells

Culturing for differentiation from human iPS cells into blood cells was carried out according to the method described in Reference 7 below, which is herein incorporated by reference. Specifically, hematopoietic progenitor cells (HPCs) were produced by culturing overnight human iPS cell colonies maintained on laminin 511-E8 (Nippi), in the presence of 50 ng/ml activin A (WAKO) and 3 µM CHIR99021 (WAKO), and co-culturing the human iPS cell colonies with C3H10T1/2 feeder cells in the presence of 20 ng/ml VEGF (manufactured by R&D SYSTEMS) for 14 days. The culture conditions were set to 37°C, 5% O₂, and 5% CO₂ for the first seven days of iPS cell maintenance and HPC induction, and 37°C, 20% O₂, and 5% CO₂ for the latter seven days.
Reference 7: Takayama N. et al., "Transient activation of c-MYC expression is critical for efficient platelet generation from human induced pluripotent stem cells", J. Exp. Med., 2010, vo.13, pages 2817-2830

### (1-2) Gene transfer system

A lentiviral vector system was used as a gene transfer system. A tetracycline controlled TET-ON^{®} gene expression induction system vector was used as a lentiviral vector. A vector into which c-MYC, BMI1, or BCL-xL is introduced under the TRE promotor was used as the vector, and the resulting vectors were respectively constructed as EN-TRE-c-Myc-Ubc-rtTA-KR, EN-TRE-BMI1-Ubc-rtTA-KR, and EN-TRE-BCL-xL-Ubc-rtTA-KR. Then, viruses containing c-MYC, BMI1, and BCL-xL vectors were prepared through gene transfer of the lentiviral vector into 293T cells (Reference 1 and Reference 8 below, which are herein incorporated by reference). Then, by infecting the cells of interest with the resulting virus, c-MYC, BMI1, and BCL-xL genes are respectively introduced into the genomic sequences of the cells of interest. Furthermore, when the c-MYC, BMI1, and BCL-xL genes have been stably introduced into the genomic sequences in the resulting cells, the c-MYC, BMI1, and BCL-xL genes can be forcibly expressed in the cells by adding doxycycline (clontech#631311) to a culture medium.
Reference 8: Yamaguchi et al., "Development of an All-in-One Inducible Lentiviral Vector for Gene Specific Analysis of Reprogramming." PLoS ONE, 2012, vol.7 (7) e41007

### (1-3) Induction of megakaryocyte cell line by introducing three genes into hematopoietic progenitor cells

HPCs produced in Example 1 (1-1) above were infected with three types of lentiviruses produced in Example 1 (1-2) to induce megakaryocyte progenitor cells. Cell lines having sufficient proliferative capability in about 25 days after the infection were immunostained using anti-human CD41a-APC antibody (manufactured by BioLegend) and anti-human CD42b-PE antibody (manufactured by eBioscience), and anti-human CD235ab-Pacific Blue (anti-CD235ab-PB; manufactured by BioLegend) antibody, and analysis was performed using FACS Verse (trademark). Cell lines having a CD41a positive rate of 50% or more and a CD42b positive rate of 50% or more were used as immortalized megakaryocyte cell lines (MKCL, equivalent to pre-multinucleated megakaryocyte cells), and were used in the following examination.

### (1-4) Proliferative culturing of megakaryocyte cell line

The obtained MKCL was proliferatively cultured in a 10-cm dish (10 mL/dish) or 125-ml shaking flask. With regard to a culture medium, IMDM (Sigma Aldrich #I3390) was used as a basal medium, and the following components were added (the concentration refers to the final concentration, a growth medium). The culture conditions were set to 37°C, 5% CO₂, and a shaking speed during shaking of 100 rpm in a shaker with a rotation diameter of 19 mm.
FBS (Hyclone #SH30071.03, fetal bovine serum) 15%,
Glutamax (GIBCO #3505-061) 2 mmol/L,
ITS-G (Gibco #41400-045) 100-fold dilution,
MTG (monothioglycerol, Fujifilm Wako Pure Chemical Corporation #195-157) 450 µmol/L, ascorbic acid (NIPRO #49871900040329) 50 µg/mL,
SCF (AGC #MEGAKARYON-SCF) 50 ng/mL,
TPO-like active substance 200 ng/mL, and
doxycycline (DOX) (clontech #631311) 1 µg/mL.

### (2) Induction of multinucleation of immortalized megakaryocytes

Next, multinucleation of immortalized megakaryocytes was induced by culturing, in a culture medium to which an AhR inhibitor, a ROCK inhibitor, a DYRK inhibitor, a myosin 2 inhibitor, and harmine were added in addition to DOX. Specifically, the immortalized megakaryocyte cell line obtained in Example 1(1) was suspended in a multinucleation culture medium below, to 3.0×10⁵ cells/ml. Then, the cells were seeded on a 6-well dish at 2 mL/well, or on a 125-mL Erlenmeyer Flask at 25 mL/flask, and cultured while being shaken at 100 rpm for 3 days. The culture conditions were set to 37°C and 5% CO₂.

### Multinucleation culture medium

FBS (Hyclone #SH30071.03) 15%
Glutamax (GIBCO #3505-061) 2 mmol/L
ITS-G (Gibco #41400-045) 100-fold dilution
MTG (monothioglycerol, Fujifilm Wako Pure Chemical Corporation #195-157) 450 µmol/L ascorbic acid (NIPRO #49871900040329) 50 µg/mL
lipid mixture 1 known composition (Sigma Aldrich #L0288-100ML) 0.75%
SCF (AGC #MEGAKARYON-SCF) 50 ng/mL
TPO-like active substance 200 ng/mL
doxycycline (clontech #631311) 1 µg/mL
aryl hydrocarbon receptor (AhR) inhibitor GNF351 (Calbiochem #182707) 500 nmol/L
ROCK inhibitor Y-39983 (Medchemexpress #MCH-HY-13300-10) 500 nmol/L
harmine (Sigma Aldrich #286044-1G) 5 µmol/L
DYRK inhibitor AZ191 (Medchem Express #HY-12277) 1 µmol/L
myosin 2 inhibitor (-)-Blebbistatin (Medchem Express #HY-13441) 10 µmol/L

### (3) Effects on multinucleation

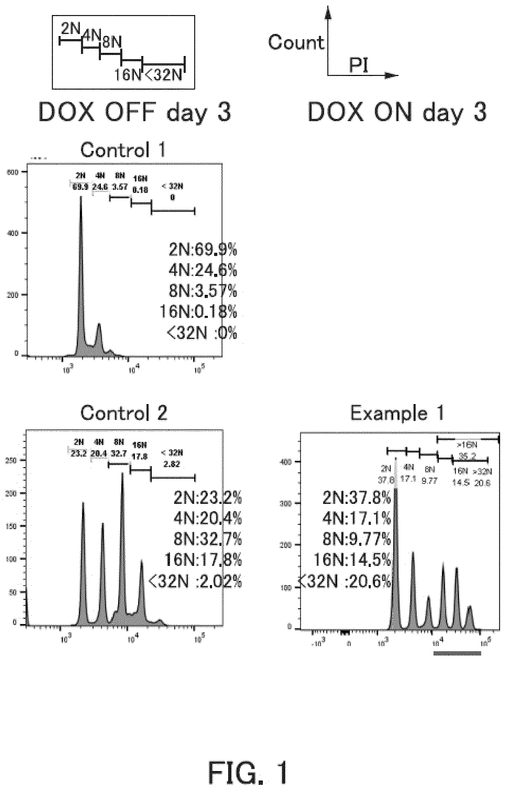

The cell suspension obtained after the culturing was collected, and the number of sets of chromosomes in the cell suspension and the size of the cells were measured using a flow cytometer. Specifically, the cell suspension was fixed at -20°C for 30 minutes to a final concentration of 70% using 100% cooled ethanol, and then was washed with PBS(-). Then, the cells were stained using a nuclear staining solution. The staining solutions were prepared by respectively adding, to PBS, 1 mg/ml of PI (propidium iodide, Sigma Aldrich #P4864-10ML) to realize a final concentration of 10 µg/mL, and 100 mg/mL of RNase (NIPPON GENE #318-06391) to realize a final concentration of 20 µg/mL.A flow cytometer was used for measurement for the stained samples. As for the obtained results, the number of sets of chromosomes (N) contained in megakaryocytes and the size of the cells was analyzed based on FSC and PI staining intensity (Example 1). A control 1 was carried out in the same manner, except that the growth medium was used instead of the multinucleation culture medium, and a control 2 was carried out in the same manner, except that a culture medium to which harmine was added to have a concentration of 10 µmol/L was used as the growth medium, instead of the multinucleation culture medium. These results are shown in FIG. 1.

FIG. 1 shows graphs of the degree of multinucleation. In FIG. 1, PI staining intensity is shown on the horizontal axis, the count number is shown on the vertical axis, and numerical values show the percentage of cells with each set of chromosomes. As shown in FIG. 1, proliferation of the immortalized megakaryocytes was maintained in the control 1, and thus multinucleated megakaryocytes were hardly detected. On the other hand, as shown in FIG. 1, when harmine was added, the immortalized megakaryocytes were multinucleated, and 32N multinucleated megakaryocytes were slightly detected. In contrast, in Example 1, multinucleation of the megakaryocytes further progressed, and not only 32N multinucleated megakaryocytes but also 64N and 128N multinucleated megakaryocytes were detected. Based on these results, it was found that, with the method for producing multinucleated megakaryocytes according to the present invention, multinucleated megakaryocytes that further multinucleated were obtained.

### (4) Production of platelets

Next, platelets were produced from multinucleated megakaryocytes by culturing the multinucleated megakaryocytes in a production culture medium below obtained by adding the following components excluding DOX to IMDM. Specifically, the multinucleated megakaryocytes obtained in Example 1(2) were washed with PBS(-) twice, and suspended in a production culture medium below, to 1.0×10⁵ cells/mL. Then, the cells were seeded on a 6-well dish at 2 mL/well, and cultured for 6 days.

### Production culture medium:

human AB serum 10% (AccessBiologicals),
Glutamax (GIBCO #3505-061) 2 mmol/L,
ITS-G (Gibco #41400-045) 100-fold dilution,
MTG (monothioglycerol, Fujifilm Wako Pure Chemical Corporation #195-157) 450 µmol/L, ascorbic acid (NIPRO #49871900040329) 50 µg/mL,
SCF (AGC #MEGAKARYON-SCF) 50 ng/mL,
TPO-like active substance 200 ng/mL,
aryl hydrocarbon receptor (AhR) inhibitor GNF351 (Calbiochem #182707) 500 nmol/L, ROCK inhibitor Y-39983 (Medchemexpress #MCH-HY-13300-10) 500 nmol/L, and enoxaparin (Sanofi) 1 U/mL.

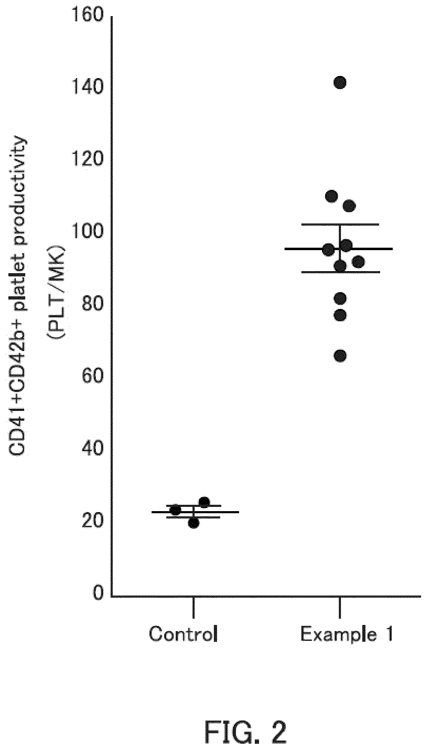

Then, as for a cell suspension containing platelets, the concentration of the platelets in the cell suspension was measured using a flow cytometer. Specifically, 180 µL of Tyrode's buffer was added to a 5-mL FACS tube, 20 µL of the cell suspension was added thereto, and then the resulting mixture was mixed. Then, staining was carried out by adding a target antibody below to each tube and allowing the tube to stand for 15 minutes. After staining, 400 µL of PBS(-) was added to the tube and mixed, 500 µL of the resulting mixture was added to TRUCOUNT TUBE^{™} and mixed, and then a flow cytometer was used for measurement. Regarding the obtained results, platelet fractions were extracted using forward scattered light (FSC) and side scattered light (SSC), the ratio of CD41⁺ CD42b⁺ fraction was obtained, and the platelet concentration was calculated based on the measured solution amount calculated by the count number of Trucount beads. The total number of platelets in the cell suspension was calculated based on the platelet concentration, and then the amount of platelets produced per megakaryocyte was calculated based on the total number of platelets and the number of seeded multinucleated megakaryocytes (Example 1). Also, a control was carried out in the same manner, except that the growth medium was used instead of the multinucleation culture medium in Example 1(2) above. These results are shown in FIG. 2.

### • Measurement of platelet concentration

0.5 µL of anti CD41 antibody APC labeling (BioLegend, Cat.No.:303710)
0.5 µL of anti CD42b antibody PE labeling (BioLegend, Cat.No.:303906)

FIG. 2 shows a graph illustrating the amount of platelets produced per megakaryocyte. In FIG. 2, the types of samples are shown on the horizontal axis, and the amount of platelets produced per megakaryocyte is shown on the vertical axis. As shown in FIG. 2, the amount of produced platelets in Example 1 was increased about four times the amount of produced platelets in the control. Based on these results, with the method for producing multinucleated megakaryocytes according to the present invention, it is possible to increase the amount of platelets produced.

### (5) Platelet production capability

Multinucleation was induced by culturing the immortalized megakaryocyte cell line obtained in Example 1(1) in the same manner as in Example 1(2). The obtained multinucleated megakaryocytes were fractionated according to the size of multinucleated megakaryocytes using bovine serum albumin. Specifically, a three-way stopcock was attached to a 50-ml injection syringe with a plunger removed, and the syringe was fixed with a clamp with a leading end of the tube facing downward. A 30% (w/v) albumin solution (derived from bovine serum, fatty acid free, Fujifilm Wako Pure Chemical Corporation #017-22231) was diluted with 1×HBSS (ThermoFisher #14175095) to prepare 2%, 4%, and 16% BSA solutions, and 16% (10 ml), 4% (4 ml), 2% (4 ml), and 0% (2 ml) solutions were layered in the above syringe in the stated order. A cell suspension containing 75×10⁵ multinucleated megakaryocyte cells prepared using 2 ml of HBSS was then applied and allowed to stand for 50 minutes, so that the cells were settled under natural gravity. Then, the three-way stopcock was loosened, and the cells were collected by dropping droplets thereof. As a result, the cell population containing the multinucleated megakaryocytes was fractionated into fractions 1 to 4 (F1 to F4). Regarding the multinucleated megakaryocytes of each fraction, the number of sets of chromosomes (N) was analyzed in the same manner as in Example 1(3) above (Example 1). Reference Example 1A was carried out in the same manner, except that a multinucleation culture medium in which Harmine, AZ191, and blebbistatin were not added was used instead of the multinucleation culture medium, and Reference Example 1B was carried in the same manner, except that a multinucleation culture medium in which harmine was not added was used instead of the multinucleation culture medium.

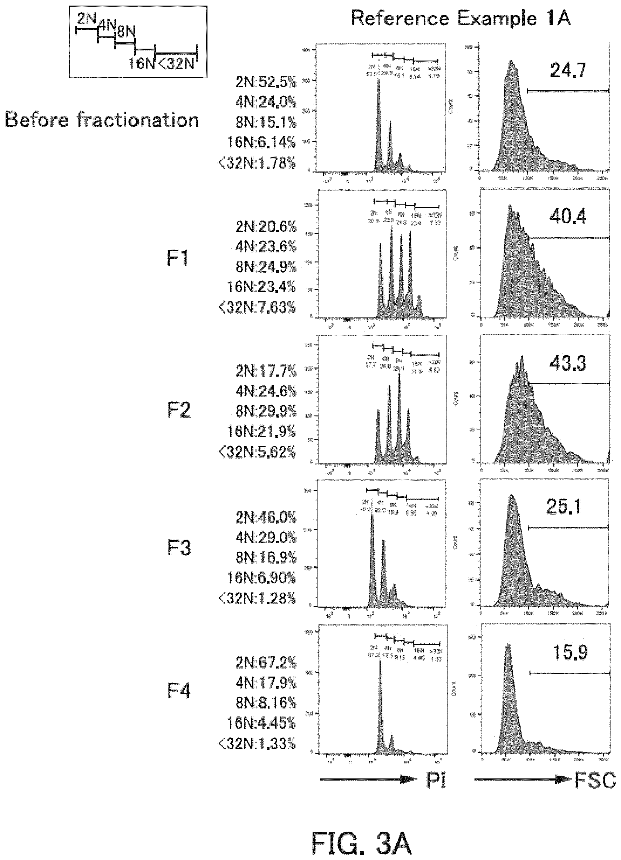

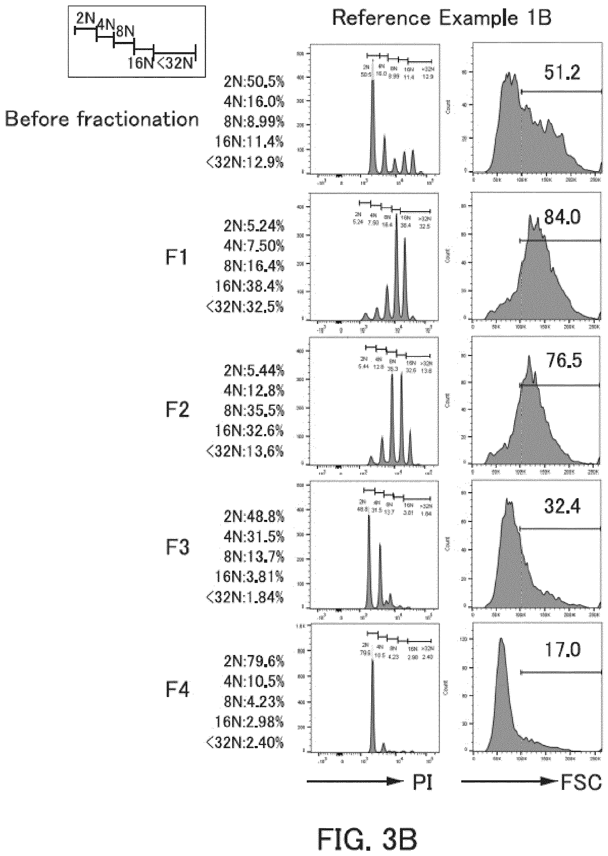

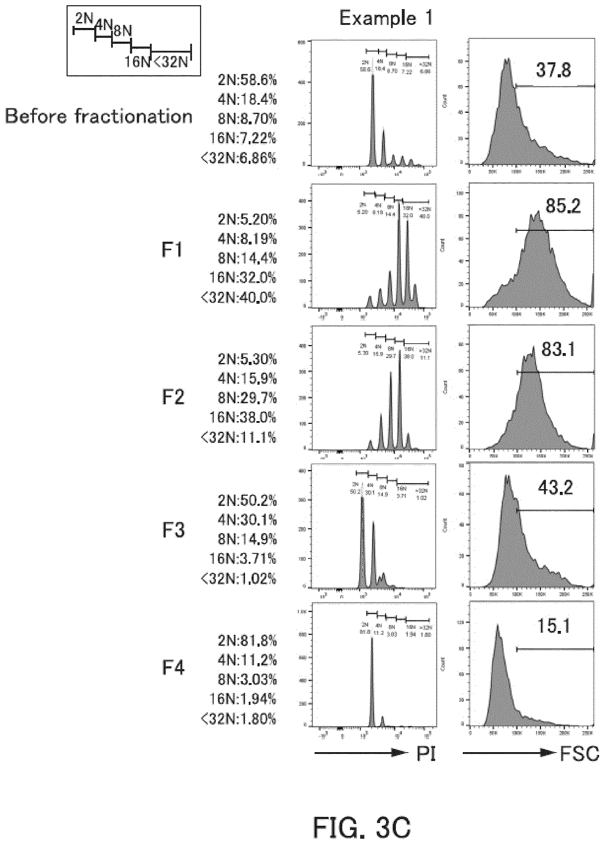

After the fractionation, platelets were produced from multinucleated megakaryocytes by culturing the multinucleated megakaryocytes in the production culture medium excluding DOX. Specifically, the multinucleated megakaryocytes of F1 to F4 were washed with PBS(-) twice, and suspended in the production culture medium, to 1.0×10⁵ cells/ml. Then, the cells were seeded on a 6-well dish at 2 mL/well, and cultured for 4 to 7 days (7 to 10 days in total). On the first day of culturing in the production culture medium, the cells in each well were observed using a phase-contrast microscope (ECLIPSE TS100 manufactured by Nikon Instec). After the culturing, regarding the cell suspension containing platelets, the amount of platelets produced per megakaryocyte was calculated in the same manner as in Example 1(4). These results are shown in FIGS. 3 to 5.

FIGS. 3A to 3C show graphs illustrating the degree of multinucleation and the size of cells. In FIGS. 3A to 3C, FIG. 3A shows the results of Reference Example 1A, FIG. 3B shows the result of Reference Example 1B, and FIG. 3C shows the result of Example 1. In FIGS. 3A to 3C, PI staining intensity or the size of cells (FSC) is shown on the horizontal axis, the count number is shown on the vertical axis, and numerical values show the percentage of cells with each set of chromosomes. As shown in FIGS. 3A to 3C, it was confirmed that, in all of Reference Example 1A, Reference Example 1B, and Example 1, the largest cells were enriched in F1, and the size of cells decreased in the order of F2, F3, and F4. Also, a comparison between Reference Example 1B and Example 1 revealed that cells had substantially the same size in all of F1 to F4.

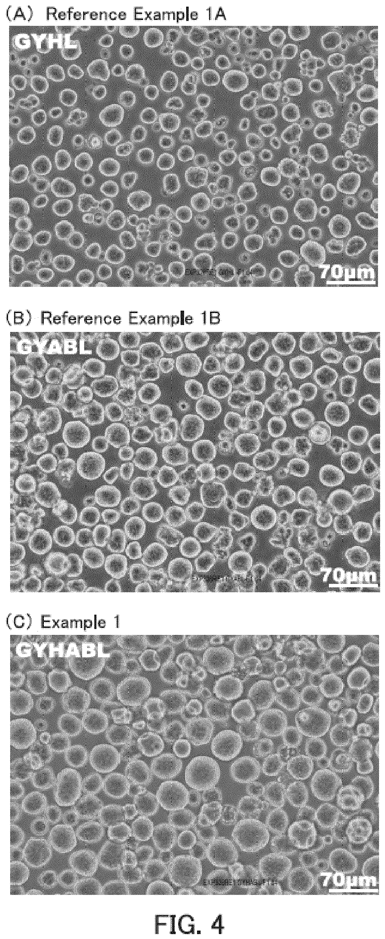

FIG. 4 shows photographs of phase-contrast images of cells in the F1 fraction. FIG. 4(A) shows the result of Reference Example 1A, FIG. 4(B) shows the result of Reference Example 1B, and FIG. 4(C) shows the result of Example 1. As shown in FIGS. 4(A) to 4(C), it was found that addition of AZ191 and blebbistatin and inhibition of DYRK and myosin 2 facilitated expansion of multinucleated megakaryocytes. On the other hand, as shown in FIGS. 4(B) and 4(C), addition of harmine had no effect on the expansion of multinucleated megakaryocytes.

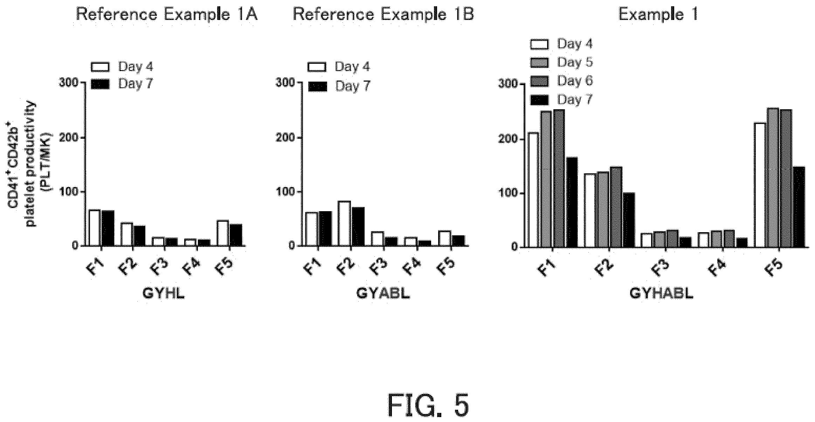

FIG. 5 shows graphs of the amount of platelets produced per megakaryocyte. In FIG. 5, the types and fractions of samples are shown on the horizontal axis, and the amount of platelets produced per megakaryocyte is shown on the vertical axis. As shown in FIG. 5, in the F1 and F2 fractions in which multinucleated megakaryocytes are particularly enriched, the amount of produced platelets in Example 1 was increased by about 2 to 3 times the amount of produced platelets in Reference Example 1A and Reference Example 1B. Therefore, it is found that it is possible to obtain multinucleated megakaryocytes with enhanced platelet production capability, using the method for producing multinucleated megakaryocytes according to the present invention. Also, as described above, the multinucleated megakaryocytes of Example 1 and the multinucleated megakaryocytes of Reference Example 1B had similar sizes. Therefore, it is inferred that the multinucleated megakaryocytes of Example 1 and the multinucleated megakaryocytes of Reference Example 1B had substantially the same maximum platelet production capability, that is, substantially the same number of platelets that can be released when all the cytoplasm is released as platelets. However, as shown in FIG. 5, the multinucleated megakaryocytes of Example 1 and the multinucleated megakaryocytes of Reference Example 1B differ from each other in the amount of produced platelets. Therefore, it was found that the multinucleated megakaryocytes of Example 1 can more efficiently release platelets, that is, the multinucleated megakaryocytes of Example 1 had more enhanced platelet production capability, compared to the multinucleated megakaryocytes of Reference Example 1B. Also, it was inferred that enhancement of the platelet production capability of the multinucleated megakaryocytes in Example 1 was caused by progression of differentiation or improved maturity of the multinucleated megakaryocyte cells, compared to the multinucleated megakaryocytes of Reference Example 1B and the like.

### Example 2

Molecules, which particularly contributed to multinucleation, were identified using the inhibitor used in Example 1.

### (1) Examination of additive agents

### (1-1) Induction of multinucleation of immortalized megakaryocytes

In five types of additive agents, an AhR inhibitor, a ROCK inhibitor, a myosin 2 inhibitor, and harmine, which were used to induce multinucleation of immortalized megakaryocytes, additive agents, which contribute to increasing the number of platelets produced, were examined. Specifically, the above examination was carried out by suspending the immortalized megakaryocyte cell line obtained in Example 1(1) in a multinucleation culture medium below, to 3.0×10⁵ cells/ml. Then, the cells were seeded on a 6-well dish at 2 mL/well, or on a 125-mL Erlenmeyer flask at 25 mL/flask, and cultured while being shaken at 100 rpm for 3 days. The culture conditions were set to 37°C and 5% CO₂. To the multinucleation culture medium, (1) all of the following inhibitors (GYHAB), (2) the inhibitors (A(-)) other than AZ191, (3) the inhibitors other than GNF351 (G(-)), (4) the inhibitors other than Y-39983 (Y(-)), (5) the inhibitors other than harmine (H(-)), (6) the inhibitors other than blebbistatin (B(-)), and (7) the inhibitors other than AZ191 and Y-39983 (GHB) were added.

### Multinucleation culture medium:

FBS (Hyclone #SH30071.03) 15%,
Glutamax (GIBCO #3505-061) 2 mmol/L,
ITS-G (Gibco #41400-045) 100-fold dilution,
MTG (monothioglycerol, Fujifilm Wako Pure Chemical Corporation #195-157) 450 µmol/L, ascorbic acid (NIPRO #49871900040329) 50 µg/mL,
SCF (AGC #MEGAKARYON-SCF) 50 ng/mL,
TPO-like active substance 200 ng/mL, and
doxycycline (clontech #631311) 1 µg/mL.

### Inhibitors:

(G) aryl hydrocarbon receptor (AhR) inhibitor GNF351 (Calbiochem #182707) 500 nmol/L,
(Y) ROCK inhibitor Y-39983 (Medchemexpress #MCH-HY-13300-10) 500 nmol/L,
(H) harmine (Sigma Aldrich #286044-1G) 5 µmol/L,
(A) DYRK inhibitor AZ 191 (Medchem Express #HY-12277) 1 µmol/L, and
(B) myosin 2 inhibitor (-)-blebbistatin (Medchem Express #HY-13441) 10 µmol/L.

### (1-2) Effects on multinucleation

The cell suspension obtained after the culturing was collected, and the number of sets of chromosomes in the cell suspension and the size of the cells were measured using a flow cytometer. Specifically, measurement was performed using the same method as in Example 1(3). As for the obtained results, the number of sets of chromosomes (N) contained in megakaryocytes and the size of the cells was analyzed based on FSC and PI staining intensity. These results are shown in FIG. 6.

FIG. 6 shows a graph illustrating the amount of platelets produced per megakaryocyte. In FIG. 6, the types of samples are shown on the horizontal axis, and the amount of platelets produced per megakaryocyte is shown on the vertical axis. As shown in FIG. 6, the amount of produced platelets did not decrease in (A(-)) in which AZ 191 was not added, as compared to that of (GYHAB) in which all of the inhibitors were added. Also, the amount of produced platelets did not decrease in (GHB) in which AZ191 and Y-39983 were not added, as compared to that of (A(-)) in which AZ 191 was not added or (GYHAB) in which all of the inhibitors were added. Also, when another inhibitor was removed, the amount of produced platelets decreased, as compared to that of the case in which all of the inhibitors were added (GYHAB). Based on the above results, it was found that, by suppressing the activities of AhR and myosin 2 and culturing cells in the presence of harmine, multinucleation of the megakaryocytes can be synergistically facilitated, and thus platelet production capability can be improved.

### Example 3

It was confirmed that multinucleation of the megakaryocytes was facilitated using the method for producing multinucleated megakaryocytes according to the present invention.

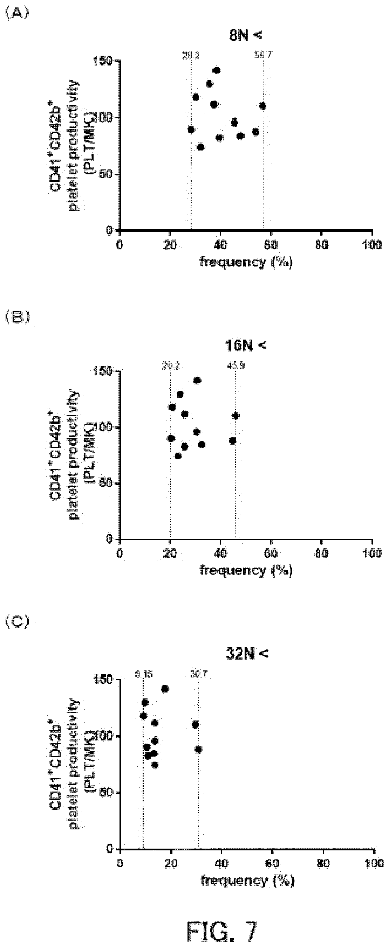

The nuclear phases of the multinucleated megakaryocytes obtained using the method for producing multinucleated megakaryocytes according to the present invention were examined. Culturing was carried out in the same manner as in Example 2(2), except that the suspension obtained by suspending the immortalized megakaryocyte cell line obtained in Example 1(1) in the multinucleation culture medium containing 15% FBS to 2.0 to 3.0×10⁵ cells/ ml was used. Regarding the resulting cell population containing the multinucleated megakaryocytes, measurement was performed using a flow cytometer in the same manner as in Example 1(3). As for the obtained results, the number of sets of chromosomes (N) contained in megakaryocytes and the size of the cells was analyzed based on FSC and PI staining intensity. A similar experiment was carried out eleven times (n=12). These results are shown in FIG. 7.

FIG. 7 shows graphs illustrating a nuclear phase of the multinucleated megakaryocytes. In FIGS. 7(A) to 7(C), the percentage of multinucleated megakaryocytes in the cell population is shown on the horizontal axis, and the amount of platelets produced per megakaryocyte is shown on the vertical axis. FIG. 7(A) shows the percentage of multinucleated megakaryocytes having a nuclear phase of 8N or more in the cell population, FIG. 7(B) shows the percentage of multinucleated megakaryocytes having a nuclear phase of 16N or more in the cell population, and FIG. 7(C) show the percentage of multinucleated megakaryocytes having a nuclear phase of 32N or more in the cell population. As shown in FIGS. 7(A) to 7(C), the percentage of multinucleated megakaryocytes having a nuclear phase of 8N or more was in a range of 28.2% to 56.7%, the percentage of multinucleated megakaryocytes having a nuclear phase of 16N or more was in a range of 20.2% to 45.9%, and the percentage of multinucleated megakaryocytes having a nuclear phase of 32N or more was in a range of 9.15% to 30.7%. Note that the percentage of multinucleated megakaryocytes having a nuclear phase of 16N or more contained in the cell population and the percentage of multinucleated megakaryocytes having a nuclear phase of 32N or more contained in the cell population were adjusted by changing the seeding density.

Based on the above, it was found that, with the method for producing multinucleated megakaryocytes according to the present invention, it was possible to prepare, in particular, a cell population having a high content of multinucleated megakaryocytes having a nuclear phase of 16N or more and a cell population having a high content of multinucleated megakaryocytes having a nuclear phase of 32N or more, that is, the multinucleation of the megakaryocytes was facilitated.

Although the present invention was described above with reference to embodiments and examples, the present invention is not limited to the above embodiments and examples. Various changes that can be understood by those skilled in the art can be made to the configuration and details of the present invention within the scope of the present invention.

This application claims priority from Japanese Patent Application No. 2021-102068 filed on June 18, 2021. The entire subject matter of the Japanese Patent Application is incorporated herein by reference.

### Supplementary Descriptions

Some or all of the above embodiments and examples may be described as, but not limited to, the following supplementary descriptions.

### Method for producing multinucleated megakaryocyte cells

### (Supplementary Description 1)

A method for producing multinucleated megakaryocyte cells with enhanced platelet production capability, the method including:
a multinucleation step of inducing multinucleated megakaryocyte cells through multinucleation of pre-multinucleated megakaryocyte cells or progenitor cells thereof in the presence of a growth factor of the pre-multinucleated megakaryocyte cells,
where in the multinucleation step, the multinucleated megakaryocyte cells are induced by suppressing the activity of at least one protein selected from the group consisting of an aryl hydrocarbon receptor (AhR), a Rho-associated kinase (ROCK), dual-specificity tyrosine phosphorylation-regulated kinases (DYRK), and myosin 2, and multinucleating the pre-multinucleated megakaryocyte cells in the presence of harmine.

### (Supplementary Description 2)

The production method according to Supplementary Description 1,
where in the multinucleation step, multinucleated megakaryocyte cells are induced through multinucleation by culturing the pre-multinucleated megakaryocyte cells or the progenitor cells thereof in the presence of harmine and at least one inhibitor selected from the group consisting of an AhR inhibitor, a ROCK inhibitor, a DYRK inhibitor, and a myosin 2 inhibitor.

### (Supplementary Description 3)

The production method according to Supplementary Description 2,
where in the multinucleation step, multinucleated megakaryocyte cells are induced through multinucleation by culturing the pre-multinucleated megakaryocyte cells or the progenitor cells thereof in the presence of harmine, the AhR inhibitor, and the myosin 2 inhibitor.

### (Supplementary Description 4)

The production method according to any one of Supplementary Descriptions 1 to 3, including
a proliferation step of proliferating the pre-multinucleated megakaryocyte cells in the presence of a growth factor of the pre-multinucleated megakaryocyte cells,
where in the proliferation step, the pre-multinucleated megakaryocyte cells are proliferated in the absence of harmine without suppressing the activities of AhR, ROCK, DYRK, and myosin 2.

### (Supplementary Description 5)

The production method according to Supplementary Description 4, where the multinucleation step is carried out after the proliferation step.

### (Supplementary Description 6)

The production method according to any one of Supplementary Descriptions 1 to 5, where in the multinucleation step, the activity of the AhR is inhibited using an AhR inhibitor.

### (Supplementary Description 7)

The production method according to any one of Supplementary Descriptions 2, 3, and 6, where the AhR inhibitor includes GNF351.

### (Supplementary Description 8)

The production method according to any one of Supplementary Descriptions 1 to 7, where in the multinucleation step, the activity of the ROCK is inhibited using a ROCK inhibitor.

### (Supplementary Description 9)

The production method according to any one of Supplementary Descriptions 2, 3, and 8, where the ROCK inhibitor includes Y-39983.

### (Supplementary Description 10)

The production method according to any one of Supplementary Descriptions 1 to 9, where in the multinucleation step, the activity of the DYRK is inhibited using a DYRK inhibitor.

### (Supplementary Description 11)

The production method according to any one of Supplementary Descriptions 2, 3, and 10, where the DYRK inhibitor includes AZ191.

### (Supplementary Description 12)

The production method according to any one of Supplementary Descriptions 1 to 11, where in the multinucleation step, the activity of the myosin 2 is inhibited using a myosin 2 inhibitor.

### (Supplementary Description 13)

The production method according to any one of Supplementary Descriptions 2, 3, and 12, where the myosin 2 inhibitor includes blebbistatin.

### (Supplementary Description 14)

The production method according to any one of Supplementary Descriptions 1 to 13,
where a cell population containing the multinucleated megakaryocyte cells is used as the multinucleated megakaryocyte cells,
the multinucleated megakaryocyte cells include multinucleated megakaryocyte cells having a nuclear phase of 16N or more, and
a percentage (number of cells) of the multinucleated megakaryocyte cells having a nuclear phase of 16N or more in the cell population containing the multinucleated megakaryocyte cells is 20% or more.

### (Supplementary Description 15)

The production method according to Supplementary Description 14, where the percentage (number of cells) of the multinucleated megakaryocyte cells having a nuclear phase of 16N or more in the cell population is in a range of 20% to 90%.

### (Supplementary Description 16)

The production method according to Supplementary Description 15, where the percentage (number of cells) of the multinucleated megakaryocyte cells having a nuclear phase of 16N or more in the cell population is in a range of 20% to 70%.

### (Supplementary Description 17)

The production method according to any one of Supplementary Descriptions 1 to 16,
where a cell population containing the multinucleated megakaryocyte cells is used as the multinucleated megakaryocyte cells,
the multinucleated megakaryocyte cells include multinucleated megakaryocytes having a nuclear phase of 8N or more, and
a percentage (number of cells) of the multinucleated megakaryocyte cells having a nuclear phase of 8N or more in the cell population is in a range of 20% to 90%.

### (Supplementary Description 18)

The production method according to Supplementary Description 16, where the percentage (number of cells) of the multinucleated megakaryocytes having a nuclear phase of 8N or more in the cell population is in a range of 25% to 60%.

### (Supplementary Description 19)

The production method according to any one of Supplementary Descriptions 1 to 17,
where a cell population containing the multinucleated megakaryocyte cells is used as the multinucleated megakaryocyte cells,
the multinucleated megakaryocyte cells include multinucleated megakaryocytes having a nuclear phase of 32N or more, and
a percentage (number of cells) of the multinucleated megakaryocytes having a nuclear phase of 32N or more in the cell population is in a range of 5% to 50%.

### (Supplementary Description 20)

The production method according to Supplementary Description 19,
where the multinucleated megakaryocyte cells include multinucleated megakaryocyte cells having a nuclear phase of 32N or more, and
the percentage (number of cells) of the multinucleated megakaryocytes having a nuclear phase of 32N or more in the cell population is in a range of 5% to 35%.

### (Supplementary Description 21)

The production method according to any one of Supplementary Descriptions 1 to 20,
where the growth factor of the pre-multinucleated megakaryocyte cells is a protein encoded by an oncogene, a Polycomb gene, and/or an apoptosis suppressor gene.

### (Supplementary Description 22)

The production method according to any one of Supplementary Descriptions 1 to 21, where the pre-multinucleated megakaryocyte cells are immortalized megakaryocytes.

### (Supplementary Description 23)

The production method according to any one of Supplementary Descriptions 1 to 22, where the pre-multinucleated megakaryocyte cells are derived from a pluripotent cell.

### (Supplementary Description 24)

The production method according to Supplementary Description 23, where the pluripotent cell is induced a pluripotent stem cell.

### (Supplementary Description 25)

The production method according to any one of Supplementary Descriptions 1 to 24, where the pre-multinucleated megakaryocyte cells are derived from a human.

### Method for producing platelets

### (Supplementary Description 26)

A method for producing platelets, including:
a multinucleation step of inducing multinucleated megakaryocyte cells from pre-multinucleated megakaryocyte cells; and
a platelet production step of producing platelets from the multinucleated megakaryocyte cells,
where the multinucleation step is carried out using the method for producing multinucleated megakaryocyte cells according to any one of Supplementary Descriptions 1 to 25.

### Method for producing platelet preparation

### (Supplementary Description 27)

A method for producing a platelet preparation, including:
a preparation step of producing a platelet preparation from platelets,
where the platelets are obtained using the method for producing platelets according to Supplementary Description 26.

### Method for producing blood preparation

### (Supplementary Description 28)

A method for producing a blood preparation, including:
a blood preparation step of producing a blood preparation by mixing platelets with another component,
where the platelets are obtained using the method for producing platelets according to Supplementary Description 26.

### Cell population containing multinucleated megakaryocyte cells

### (Supplementary Description 29)

A cell population containing multinucleated megakaryocytes,
where the multinucleated megakaryocytes include multinucleated megakaryocytes having a nuclear phase of 16N or more, and
a percentage (number of cells) of the multinucleated megakaryocytes having a nuclear phase of 16N or more in the cell population is 20% or more.

### (Supplementary Description 30)

The cell population according to Supplementary Description 29, where the percentage (number of cells) of the multinucleated megakaryocytes having a nuclear phase of 16N or more in the cell population is in a range of 20% to 90%.

### (Supplementary Description 31)

The cell population according to Supplementary Description 30, where the percentage (number of cells) of the multinucleated megakaryocytes having a nuclear phase of 16N or more in the cell population is in a range of 20% to 70%.

### (Supplementary Description 32)

The cell population according to any one of Supplementary Descriptions 29 to 31,
where the multinucleated megakaryocytes include multinucleated megakaryocytes having a nuclear phase of 8N or more, and
a percentage (number of cells) of the multinucleated megakaryocytes having a nuclear phase of 8N or more in the cell population is in a range of 20% to 90%.

### (Supplementary Description 33)

The cell population according to Supplementary Description 32,
where the percentage (number of cells) of the multinucleated megakaryocytes having a nuclear phase of 8N or more in the cell population is in a range of 25% to 60%.

### (Supplementary Description 34)

The cell population according to any one of Supplementary Descriptions 29 to 33,
where the multinucleated megakaryocytes include multinucleated megakaryocytes having a nuclear phase of 32N or more, and
a percentage (number of cells) of the multinucleated megakaryocytes having a nuclear phase of 32N or more in the cell population is in a range of 5% to 50%.

### (Supplementary Description 35)

The cell population according to Supplementary Description 34,
where the multinucleated megakaryocytes include multinucleated megakaryocytes having a nuclear phase of 32N or more, and
the percentage (number of cells) of the multinucleated megakaryocytes having a nuclear phase of 32N or more in the cell population is in a range of 5% to 35%.

### (Supplementary Description 36)

A cell population containing multinucleated megakaryocytes,
where the multinucleated megakaryocytes include multinucleated megakaryocytes having a nuclear phase of 32N or more, and
a percentage (number of cells) of the multinucleated megakaryocytes having a nuclear phase of 32N or more in the cell population is in a range of 5% to 50%.

### (Supplementary Description 37)

The cell population according to Supplementary Description 36,
where the multinucleated megakaryocytes include multinucleated megakaryocytes having a nuclear phase of 32N or more, and
the percentage (number of cells) of the multinucleated megakaryocytes having a nuclear phase of 32N or more in the cell population is in a range of 5% to 35%.

### (Supplementary Description 38)

The cell population according to Supplementary Description 36 or 37,
where the multinucleated megakaryocytes include multinucleated megakaryocytes having a nuclear phase of 8N or more, and
a percentage (number of cells) of the multinucleated megakaryocytes having a nuclear phase of 8N or more in the cell population is in a range of 20% to 70%.

### (Supplementary Description 39)

The cell population according to Supplementary Description 38, where a percentage (number of cells) of the multinucleated megakaryocytes having a nuclear phase of 8N or more in the cell population is in a range of 25% to 60%.

### (Supplementary Description 40)

The cell population according to any one of Supplementary Descriptions 29 to 39, where the pre-multinucleated megakaryocyte cells are immortalized megakaryocytes.

### (Supplementary Description 41)

The cell population according to any one of Supplementary Descriptions 29 to 40, where the pre-multinucleated megakaryocyte cells are derived from a pluripotent cell.

### (Supplementary Description 42)

The cell population according to any one of Supplementary Descriptions 29 to 41, where the pre-multinucleated megakaryocyte cells contain an exogenous oncogene, an exogenous Polycomb gene, and/or an exogenous apoptosis suppressor gene.

### Industrial Applicability

As described above, according to the present invention, it is possible to produce multinucleated megakaryocytes with enhanced platelet production capability. Thus, according to the present invention, for example, it is possible to improve the amount of platelets produced. Therefore, the present invention is extremely useful, for example, in the field of cell medicine, medical field, and the like where platelets are used.

## Claims

1. A method for producing multinucleated megakaryocyte cells with enhanced platelet production capability, the method comprising:
a multinucleation step of inducing multinucleated megakaryocyte cells through multinucleation of pre-multinucleated megakaryocyte cells or progenitor cells thereof in the presence of a growth factor of the pre-multinucleated megakaryocyte cells,
wherein in the multinucleation step, the multinucleated megakaryocyte cells are induced by suppressing the activity of at least one protein selected from the group consisting of an aryl hydrocarbon receptor (AhR), a Rho-associated kinase (ROCK), a dual-specificity tyrosine phosphorylation-regulated kinase (DYRK), and myosin 2, and multinucleating the pre-multinucleated megakaryocyte cells in the presence of harmine.

2. The production method according to claim 1,
wherein in the multinucleation step, the multinucleated megakaryocyte cells are induced through multinucleation by culturing the pre-multinucleated megakaryocyte cells or the progenitor cells thereof in the presence of harmine and at least one inhibitor selected from the group consisting of an AhR inhibitor, a ROCK inhibitor, a DYRK inhibitor, and a myosin 2 inhibitor.

3. The production method according to claim 2,
wherein in the multinucleation step, the multinucleated megakaryocyte cells are induced through multinucleation by culturing the pre-multinucleated megakaryocyte cells or the progenitor cells thereof in the presence of harmine, the AhR inhibitor, and the myosin 2 inhibitor.

4. The production method according to any one of claims 1 to 3, comprising:
a proliferation step of proliferating the pre-multinucleated megakaryocyte cells in the presence of a growth factor of the pre-multinucleated megakaryocyte cells,
wherein in the proliferation step, the pre-multinucleated megakaryocyte cells are proliferated in the absence of harmine without suppressing the activities of the AhR, the ROCK, the DYRK, and the myosin 2.

5. The production method according to claim 4,
wherein the multinucleation step is carried out after the proliferation step.

6. The production method according to any one of claims 1 to 5,
wherein in the multinucleation step, the activity of the AhR is inhibited using an AhR inhibitor.

7. The production method according to any one of claims 2, 3, and 6,
wherein the AhR inhibitor includes GNF351.

8. The production method according to any one of claims 1 to 7,
wherein in the multinucleation step, the activity of the ROCK is inhibited using a ROCK inhibitor.

9. The production method according to any one of claims 2, 3, and 8,
wherein the ROCK inhibitor includes Y-39983.

10. The production method according to any one of claims 1 to 9,
wherein in the multinucleation step, the activity of the myosin 2 is inhibited using a myosin 2 inhibitor.

11. The production method according to any one of claims 2, 3, and 10,
wherein the myosin 2 inhibitor includes blebbistatin.

12. The production method according to any one of claims 1 to 11,
wherein a cell population comprising the multinucleated megakaryocyte cells is used as the multinucleated megakaryocyte cells,
the multinucleated megakaryocyte cells comprise multinucleated megakaryocyte cells having a nuclear phase of 16N or more, and
a percentage (number of cells) of the multinucleated megakaryocyte cells having a nuclear phase of 16N or more in the cell population containing the multinucleated megakaryocyte cells is in a range of 20% or more.

13. The production method according to claim 12,
wherein the percentage (number of cells) of the multinucleated megakaryocyte cells having a nuclear phase of 16N or more in the cell population is in a range from 20% to 90%.

14. The production method according to claim 13,
wherein the percentage (number of cells) of the multinucleated megakaryocyte cells having a nuclear phase of 16N or more in the cell population is in a range from 20% to 70%.

15. The production method according to any one of claims 1 to 14,
wherein a cell population comprising the multinucleated megakaryocyte cells is used as the multinucleated megakaryocyte cells,
the multinucleated megakaryocyte cells comprise multinucleated megakaryocytes having a nuclear phase of 32N or more, and
a percentage (number of cells) of the multinucleated megakaryocytes having a nuclear phase of 32N or more in the cell population is in a range from 5% to 50%.

16. The production method according to claim 15,
wherein the multinucleated megakaryocyte cells comprise multinucleated megakaryocytes having a nuclear phase of 32N or more, and
the percentage (number of cells) of the multinucleated megakaryocytes having a nuclear phase of 32N or more in the cell population is in a range from 5% to 35%.

17. The production method according to any one of claims 1 to 16,
wherein the growth factor of the pre-multinucleated megakaryocyte cells is a protein encoded by an oncogene, a Polycomb gene, and/or an apoptosis suppressor gene.

18. The production method according to any one of claims 1 to 17,
wherein the pre-multinucleated megakaryocyte cells are immortalized megakaryocytes.

19. A method for producing platelets, comprising:
a multinucleation step of inducing multinucleated megakaryocyte cells from pre-multinucleated megakaryocyte cells; and
a platelet production step of producing platelets from the multinucleated megakaryocyte cells,
wherein the multinucleation step is carried out using the method for producing multinucleated megakaryocyte cells according to any one of claims 1 to 18.

20. A method for producing a platelet preparation, comprising:
a preparation step of producing a platelet preparation from platelets,
wherein the platelets are obtained using the method for producing platelets according to claim 19.

21. A method for producing a blood preparation, comprising:
a blood preparation step of producing a blood preparation by mixing platelets with another component,
wherein the platelets are obtained using the method for producing platelets according to claim 19.

22. A cell population comprising multinucleated megakaryocytes,
wherein the multinucleated megakaryocytes comprise multinucleated megakaryocytes having a nuclear phase of 16N or more, and
a percentage (number of cells) of the multinucleated megakaryocytes having a nuclear phase of 16N or more in the cell population is in a range of 20% or more.

23. The cell population according to claim 22,
wherein the percentage (number of cells) of the multinucleated megakaryocytes having a nuclear phase of 16N or more in the cell population is in a range from 20% to 90%.

24. The cell population according to claim 23,
wherein the percentage (number of cells) of the multinucleated megakaryocytes having a nuclear phase of 16N or more in the cell population is in a range from 20% to 70%.

25. The cell population according to any one of claims 22 to 24,
wherein the multinucleated megakaryocytes comprise multinucleated megakaryocytes having a nuclear phase of 8N or more, and
a percentage (number of cells) of the multinucleated megakaryocytes having a nuclear phase of 8N or more in the cell population is in a range from 20% to 90%.

26. The cell population according to claim 25,
wherein the percentage (number of cells) of the multinucleated megakaryocytes having a nuclear phase of 8N or more in the cell population is in a range from 25% to 60%.

27. The cell population according to any one of claims 22 to 26,
wherein the multinucleated megakaryocytes comprise multinucleated megakaryocytes having a nuclear phase of 32N or more, and
a percentage (number of cells) of the multinucleated megakaryocytes having a nuclear phase of 32N or more in the cell population is in a range from 5% to 50%.

28. The cell population according to claim 27,
wherein the multinucleated megakaryocytes comprise multinucleated megakaryocytes having a nuclear phase of 32N or more, and
the percentage (number of cells) of the multinucleated megakaryocytes having a nuclear phase of 32N or more in the cell population is in a range from 5% to 35%.

29. The cell population according to any one of claims 22 to 28,
wherein the pre-multinucleated megakaryocyte cells are immortalized megakaryocytes.

30. The cell population according to any one of claims 22 to 29,
wherein the pre-multinucleated megakaryocyte cells comprise an exogenous oncogene, an exogenous Polycomb gene, and/or an exogenous apoptosis suppressor gene.
